# EUROPEAN PATENT SPECIFICATION

(11) **EP 4 186 416 B1**
(45) Date of publication and mention of the grant of the patent: **09.09.2026**
(21) Application number: 21848644.7
(22) Date of filing: 26.07.2021
(51) Int. Cl.: A61B 5/024

(54) **METHOD FOR ACQUIRING HEART RATE AND ELECTRONIC DEVICE**
VERFAHREN ZUR ERFASSUNG DER HERZFREQUENZ UND ELEKTRONISCHE VORRICHTUNG
PROCÉDÉ D'ACQUISITION DE FRÉQUENCE CARDIAQUE ET DISPOSITIF ÉLECTRONIQUE

(30) Priority: 28.07.2020 CN 202010740968
(43) Date of publication of application: 31.05.2023
(73) Proprietor: Huawei Technologies Co., Ltd., Shenzhen, Guangdong 518129 (CN)
(72) Inventor: YANG, Sulin, Shenzhen, Guangdong 518129 (CN)
(74) Representative: MERH-IP Matias Erny Reichl Hoffmann Patentanwälte PartG mbB
(86) International application number: PCT/CN2021/108481
(87) International publication number: WO 2022/022469

(56) References cited:
- WO-A1-2009/071128
- WO-A1-2020/101249
- CN-A- 105 592 780
- CN-A- 105 916 436
- CN-A- 109 152 530
- CN-A- 110 461 226
- JP-A- H1 099 303
- US-A1- 2011 060 200
- US-A1- 2016 296 174
- US-A1- 2017 311 825
- US-A1- 2018 146 870

## Description

### TECHNICAL FIELD

This application relates to the field of terminal technologies, and in particular, to a method for obtaining a heart rate and an electronic device.

### BACKGROUND

A photoplethysmography (photoplethysmography, PPG) is a non-invasive detection manner of detecting a volume change of blood in a blood vessel based on a photoelectric method. Currently, an electronic device may use the PPG to detect a heart rate and blood oxygen saturation of a user. The electronic device may include an optical transmitter and an optical receiver. The electronic device may be worn on a wrist, an abdomen, a leg, or another location of the user, so that the optical transmitter can be close to a skin of the user. The optical transmitter may transmit an optical signal to a skin surface. The optical receiver may receive a reflected or scattered optical signal, and generate a PPG signal. The transmitted optical signal may be absorbed, reflected, and scattered by tissues, such as blood in the blood vessel, a skin, a muscle, and a skeleton. A volume of the blood in the blood vessel changes during relaxation and contraction of the heart, and an optical signal absorbed by the blood in the blood vessel also changes. Strength of the optical signal received by the optical receiver also varies with the contraction and relaxation of the heart. The electronic device can implement heart rate detection based on a change of the strength of the optical signal received by the optical receiver.

Based on the foregoing PPG-based method for detecting a heart rate, an accurate detection result can be provided when the electronic device and the skin of the user are kept relatively still. However, if the user is in a motion state, the PPG signal generated by the optical receiver may include motion noise, which affects accuracy of a detection result. A reason for the motion noise may be, for example, that the user sweats during motion, and sweat affects the strength of the optical signal received by the optical receiver. Alternatively, during motion of the user, relative movement generated between the electronic device and the skin of the user affects the strength of the optical signal received by the optical receiver.

Therefore, how to reduce noise in the PPG signal is an issue that needs to be urgently addressed at present when the electronic device obtains the heart rate.

Document WO 2020/101249A1 relates an electronic device which includes a photoplethysmogram sensor configured to measure a biometric signal from a body part of a user while being in contact with the body part of the user, and a memory which stores instructions, when executed, to allow the processor to receive data from the photoplethysmogram sensor and determine a distance between the body part of the user and the fastening structure.

Dcoment JP H1099303A relates a photoplethysmographic measurement device. Signals from multiple light emitters are combined into a single multiplexed light signal in a test unit before being delivered to a physically separated probe head attached to a test subject. The probe then causes the single multiplexed signal to be transmitted through a tissue under test on the test subject, after which it is processed to determine a blood analyte level of the test subject. By combining the light signals into a single multiplexed signal before delivering the signals to the probe, a single light guide, such as a single optical fibre, is used to deliver the signals to the probe.

Document US 2018/146870A1 relates to a menthod in which biopotential waveforms such as ERPs, EEGs, ECGs, or EMGs are classified accurately by dynamically fusing classification information from multiple electrodes, tests, or other data sources. These different data sources or "channels" are ranked at different time instants according to their respective univariate classification accuracies. Channel rankings are determined during training phase in which classification accuracy of each channel at each time-instant is determined. Classifiers are simple univariate classifiers which only require univariate parameter estimation. Using classification information, a rule is formulated to dynamically select different channels at different time-instants during the testing phase. Independent decisions of selected channels at different time instants are fused into a decision fusion vector. The resulting decision fusion vector is optimally classified using a discrete Bayes classifier.

### SUMMARY

This application provides a method for obtaining a heart rate and an electronic device. The present invention is defined by the appended claims. In the following, parts of the description and drawings referring to former embodiments which do not necessarily comprise all features to implement embodiments of the claimed invention are understood to not represent embodiments of the invention but to relate to be examples useful for understanding the embodiments of the invention. The electronic device includes a first optical transmitter, a second optical transmitter, a first optical receiver, and a second optical receiver. The first optical transmitter and the second optical transmitter may be respectively configured to transmit a first optical signal and a second optical signal with different wavelengths. The first optical receiver may receive a reflected or scattered first optical signal, and generate a first PPG signal. The second optical receiver may receive a reflected or scattered second optical signal, and generate a second PPG signal. The second PPG signal may be used to determine a magnitude of noise in the first PPG signal. The electronic device can reduce the noise in the first PPG signal by using the second PPG signal, and obtain a heart rate based on a noise-reduced first PPG signal. Therefore, the electronic device can reduce the noise in the PPG signal, improving accuracy of heart rate detection.

According to a first aspect, an embodiment of this application provides a method for obtaining a heart rate, and the method is applied to an electronic device. The electronic device includes a bottom wall, at least one first optical transmitter, at least one second optical transmitter, at least one first optical receiver, and at least one second optical receiver. A first opening and a second opening are provided on the bottom wall. Optical signals transmitted by the first optical transmitter and the second optical transmitter are transmitted from the bottom wall through the first opening, and the first optical receiver and the second optical receiver receive optical signals through the second opening. The first optical receiver includes a first optical filter, and the second optical receiver includes a second optical filter. The method includes: The electronic device drives the first optical transmitter to transmit a first optical signal with a first wavelength, and drive the second optical transmitter to transmit a second optical signal with a second wavelength. The first wavelength is greater than or equal to 492 nanometers and less than or equal to 577 nanometers. The second wavelength is greater than or equal to 1000 nanometers and less than or equal to 1500 nanometers. The electronic device receives a third optical signal by using the first optical receiver, and obtain a first PPG signal based on the third optical signal. The third optical signal is formed through reflection or scattering of the first optical signal. The first optical filter in the first optical receiver filters reflection or scattering of the second optical signal. The electronic device receives a fourth optical signal by using the second optical receiver, and obtain a second PPG signal based on the fourth optical signal. The fourth optical signal is formed through reflection or scattering of the second optical signal. The first optical filter in the first optical receiver filters reflection or scattering of the second optical signal. The electronic device obtains a third PPG signal by performing subtraction processing on the first PPG signal and the second PPG signal. The electronic device obtains a heart rate by using the third PPG signal.

In this application, the electronic device may be worn on a wrist, an abdomen, a leg, or another location of a user. The bottom wall of the electronic device comes into contact with a skin of a human body when the electronic device is worn. Therefore, when the electronic device is worn, the first optical signal transmitted by the first optical transmitter and the second optical signal transmitted by the second optical transmitter may be transmitted to the skin of the human body from the opening of the bottom wall. The first optical signal and the second optical signal may penetrate the skin of the human body to a depth of tens of microns to several millimeters. The first optical signal and the second optical signal may be absorbed, reflected, and scattered by human tissues, such as blood, a skin, a muscle, and a skeleton.

In this application, both the first PPG signal and the second PPG signal are current signals. Each of the first PPG signal and the second PPG signal includes a direct current signal and an alternating current signal, and may further include motion noise caused by a user motion and random noise generated by the electronic device during signal processing. The alternating current signal in the first PPG signal may be used to calculate the heart rate. The direct current signal in the first PPG signal may be filtered out by a filter in the electronic device. Because of the motion noise and the random noise, accuracy of the heart rate obtained by the electronic device by using the alternating current signal in the first PPG signal is low.

Blood in a blood vessel has higher absorptivity for the first optical signal than for the second optical signal. Therefore, the first optical signal is more sensitive to a volume change of the blood in the blood vessel than the second optical signal. Strength of the alternating current signal in the first PPG signal is far higher than strength of the alternating current signal in the second PPG signal. Further, the user may have same or similar impact on the first optical signal and the second optical signal during motion, and the motion noise in the first PPG signal and the second PPG signal may be the same or similar. The electronic device can reduce the motion noise in the first PPG signal by using the second PPG signal.

With reference to the first aspect, in a possible implementation, the electronic device may first convert the first PPG signal and the second PPG signal into a fourth PPG signal and a fifth PPG signal respectively, and then perform an operation of subtracting the fifth PPG signal from the fourth PPG signal to obtain the third PPG signal. The third PPG signal, the fourth PPG signal, and the fifth PPG signal are voltage signals. The third PPG signal may be equivalent to a signal in which motion noise and random noise are reduced. The electronic device may obtain the heart rate by using the third PPG signal, improving accuracy of heart rate detection.

Specifically, by using a trans-impedance amplifier, the electronic device may convert the first PPG signal into the fourth PPG signal, and convert the second PPG signal into the fifth PPG signal. After obtaining the third PPG signal, the electronic device may perform one or more of the following processing on the third PPG signal by using a signal conditioner: amplification, filtering, sampling, and analog-to-digital conversion. Alternatively, the electronic device may first perform one or more of the following processing on the fourth PPG signal and the fifth PPG signal by using the signal conditioner: amplification, filtering, sampling, and analog-to-digital conversion. Then, the electronic device may perform, by using a digital signal processor, a subtraction operation on the fourth PPG signal and the fifth PPG signal that are processed by the signal conditioner, to obtain the third PPG signal.

With reference to the first aspect, in another possible implementation, the first optical receiver and the second optical receiver in the electronic device may be connected in series. When the first optical receiver generates the first PPG signal, some current signals in the first PPG signal may flow to the second optical receiver. Strength of the some current signals flowing to the second optical receiver may be the same as strength of the second PPG signal generated by the second optical receiver. A current signal in the first PPG signal that does not flow to the second optical receiver is a sixth PPG signal. The sixth PPG signal may be equivalent to a signal in which motion noise and random noise are reduced. The electronic device may convert the sixth PPG signal into a voltage signal, to obtain the third PPG signal.

When optical paths of the first optical signal and the second optical signal are the same or similar to each other in a transmission process, the user motion may have same or similar impact on the first optical signal and the second optical signal. The electronic device may set the optical paths of the first optical signal and the second optical signal, to improve accuracy of heart rate detection.

With reference to the first aspect, in a possible implementation, the electronic device may perform multiplexing processing on the first optical signal and the second optical signal by using a wavelength division multiplexer.

Specifically, the electronic device may include the wavelength division multiplexer and a reflector. The wavelength division multiplexer may include a first light incident surface, a second light incident surface, and a first light emergent surface. The first light incident surface may be opposite to a light emergent surface of the first optical transmitter. The second light incident surface may be opposite to a light emergent surface of the second optical transmitter.

The electronic device may drive the first optical transmitter to transmit the first optical signal to the first light incident surface, and drive the second optical transmitter to transmit the second optical signal to the second light incident surface. The first optical signal and the second optical signal may be received by the wavelength division multiplexer. The electronic device may perform multiplexing processing on the first optical signal and the second optical signal by using the wavelength division multiplexer, to obtain a fifth optical signal. The fifth optical signal may be transmitted from the first light emergent surface to the reflector, and transmitted from the bottom wall of the electronic device after being reflected by the reflector.

The fifth optical signal may include the first optical signal and the second optical signal. The fifth optical signal may be received by the first optical receiver and the second optical receiver after being reflected or scattered. The first optical receiver and the second optical receiver may include optical filters. The first optical receiver may filter out the second optical signal from the received fifth optical signal by using the optical filter, to obtain the first optical signal and further generate the first PPG signal. The second optical receiver may filter out the first optical signal from the received fifth optical signal by using the optical filter, to obtain the second optical signal and further generate the second PPG signal.

Optionally, the electronic device may transmit the first optical signal and the second optical signal by using a waveguide, to reduce a loss of the optical signal in the transmission process. Specifically, the electronic device may include a first waveguide, a second waveguide, and a third waveguide. The first waveguide may be configured to connect the light emergent surface of the first optical transmitter to the first light incident surface. The second waveguide may be configured to connect the light emergent surface of the second optical transmitter to the second light incident surface. The third waveguide may be configured to connect the first light emergent surface to the reflector.

The electronic device may transmit the first optical signal from the first optical transmitter to the wavelength division multiplexer by using the first waveguide. The electronic device may transmit the second optical signal from the second optical transmitter to the wavelength division multiplexer by using the second waveguide. The electronic device may transmit the fifth optical signal from the wavelength division multiplexer to the reflector by using the third waveguide.

With reference to the first aspect, in a possible implementation, the first optical receiver and the second optical receiver may be a same optical receiver. The electronic device may time-divisionally drive the first optical transmitter to transmit the first optical signal and drive the second optical transmitter to transmit the second optical signal.

Specifically, the electronic device may drive, in a first time period, the first optical transmitter to transmit the first optical signal. The electronic device may obtain the first PPG signal based on the third optical signal received by the optical receiver. The third optical signal may be formed through reflection or scattering of the first optical signal.

The electronic device may drive, in a second time period, the second optical transmitter to transmit the second optical signal. The electronic device may obtain the second PPG signal based on the fourth optical signal received by the optical receiver. The fourth optical signal may be formed through reflection or scattering of the second optical signal. An interval between the first time period and the second time period is less than first duration. A value of the first duration may approach zero.

According to a second aspect, an embodiment of this application provides an electronic device, and the electronic device includes a bottom wall, at least one first optical transmitter, at least one second optical transmitter, at least one first optical receiver, at least one second optical receiver, at least one second optical receiver, an analog front end, and a processor. A first opening and a second opening are provided on the bottom wall. Optical signals transmitted by the first optical transmitter and the second optical transmitter are transmitted from the bottom wall through the first opening. The first optical receiver and the second optical receiver receive optical signals through the second opening, the first optical receiver (212A) includes a first optical filter, the second optical receiver (212B) includes a second optical filter.

The first optical transmitter is configured to transmit a first optical signal with a first wavelength. The first wavelength is greater than or equal to 492 nanometers and less than or equal to 577 nanometers. The second optical transmitter is configured to transmit a second optical signal with a second wavelength. The second wavelength is greater than or equal to 1000 nanometers and less than or equal to 1500 nanometers. The first optical receiver is configured to receive a third optical signal, and generate a first PPG signal based on the third optical signal. The third optical signal is formed through reflection or scattering of the first optical signal. The first optical filter in the first optical receiver filters reflection or scattering of the second optical signal. The second optical receiver is configured to receive a fourth optical signal, and generate a second PPG signal based on the fourth optical signal. The fourth optical signal is formed through reflection or scattering of the second optical signal. The second optical filter in the second optical receiver filters reflection or scattering of the first optical signal.

The analog front end is configured to obtain a third PPG signal by subtraction processing on the first PPG signal and the second PPG signal.

The processor is configured to obtain a heart rate by using the third PPG signal.

In this application, the analog front end may separately establish communication connections to the first optical transmitter, the second optical transmitter, the first optical receiver, the second optical receiver, and the processor. The analog front end may include a light source driver. When receiving an instruction that comes from the processor and that is used to instruct the first optical transmitter and the second optical transmitter to transmit optical signals, the light source driver in the analog front end may drive the first optical transmitter to transmit the first optical signal, and drive the second optical transmitter to transmit the second optical signal.

The analog front end may further include a trans-impedance amplifier. Both the first PPG signal and the second PPG signal are current signals. When receiving the first PPG signal and the second PPG signal, the analog front end may convert the first PPG signal into a fourth PPG signal and convert the second PPG signal into a fifth PPG signal by using the trans-impedance amplifier. Both the fourth PPG signal and the fifth PPG signal are voltage signals. The analog front end may further include one or more of the following: a calculator, a signal conditioner, and a digital signal processor. Both the calculator and the digital signal processor may perform subtraction processing on the fourth PPG signal and the fifth PPG signal. The signal conditioner may be configured to perform one or more of the following processing on a signal: amplification, filtering, sampling, and analog-to-digital conversion.

When obtaining the third PPG signal, the analog front end may send the third PPG signal to the processor. The processor may obtain the heart rate by using the third PPG signal.

With reference to the second aspect, in a possible implementation, the first optical receiver and the second optical receiver may be connected in series. Some current signals in the first PPG signal generated by the first optical receiver may flow to the second optical receiver. Strength of the some current signals flowing to the second optical receiver may be the same as strength of the second PPG signal generated by the second optical receiver. The first optical receiver may send a sixth PPG signal to the analog front end. Strength of the sixth PPG signal may be the same as strength of a current signal that is in the first PPG signal and that does not flow to the second optical receiver. Further, the analog front end may convert the sixth PPG signal into a voltage signal by using the trans-impedance amplifier, to obtain the third PPG signal.

With reference to the second aspect, in a possible implementation, the electronic device may perform multiplexing processing on the first optical signal and the second optical signal by using a wavelength division multiplexer, so that optical paths of the first optical signal and the second optical signal are consistent with each other in a transmission process.

Specifically, the electronic device may include the wavelength division multiplexer and a reflector. The wavelength division multiplexer may include a first light incident surface, a second light incident surface, and a first light emergent surface. The first light incident surface may be opposite to a light emergent surface of the first optical transmitter, and may be configured to receive an optical signal transmitted by the first optical transmitter. The second light incident surface may be opposite to a light emergent surface of the second optical transmitter, and may be configured to receive an optical signal transmitted by the second optical transmitter. The reflector may be configured to receive an optical signal transmitted from the first light emergent surface, and transmit, through the bottom wall, the optical signal transmitted from the first light emergent surface.

Optionally, the electronic device may transmit the first optical signal and the second optical signal by using a waveguide.

Specifically, the electronic device may include a first waveguide, a second waveguide, and a third waveguide. The first waveguide may be configured to connect the light emergent surface of the first optical transmitter to the first light incident surface, to transmit the first optical signal to the wavelength division multiplexer. The second waveguide may be configured to connect the light emergent surface of the second optical transmitter to the second light incident surface, to transmit the second optical signal to the wavelength division multiplexer. The third waveguide may be configured to connect the first light emergent surface to the reflector, to transmit, to the reflector, the optical signal transmitted from the first light emergent surface.

According to a third aspect, an embodiment of this application further provides another method for obtaining a heart rate. The method is applied to an electronic device. The electronic device may include: at least one first optical transmitter, a first receiver, a second receiver, a third receiver, a fourth receiver, a first accelerometer, and a second accelerometer. The first optical transmitter, the first receiver, the second receiver, the third receiver, and the fourth receiver may be located on one straight line. The first receiver and the second receiver may be distributed on one side of the first optical transmitter. The third receiver and the fourth receiver may be distributed on the other side of the first optical transmitter. A distance between the second receiver and the first optical transmitter may be greater than a distance between the first receiver and the first optical transmitter. A distance between the fourth receiver and the first optical transmitter may be greater than a distance between the third receiver and the first optical transmitter. A spacing between the first accelerometer and the fourth receiver may be less than a first spacing. A spacing between the second accelerometer and the second receiver may be less than a second spacing.

The method may include: The electronic device may drive the first optical transmitter to transmit a first optical signal with a first wavelength. The first wavelength may be greater than or equal to 492 nanometers and less than or equal to 577 nanometers. The electronic device may respectively obtain a first signal, a second signal, a third signal, and a fourth signal based on a first optical signal received by the first receiver, the second receiver, the third receiver, and the fourth receiver. The electronic device may measure a first acceleration of the electronic device in a first direction by using the first accelerometer, and measure a second acceleration of the electronic device in the first direction by using the second accelerometer. The electronic device may compare magnitudes of the first acceleration and the second acceleration, and select at least one signal from the first signal, the second signal, the third signal, and the fourth signal. The electronic device may obtain a heart rate by using the selected at least one signal.

Therefore, the electronic device may determine, by using the first accelerometer and the second accelerometer, whether there is a deflection angle between a bottom wall of the electronic device and a plane on which a skin of a user is located. When it is determined that there is the deflection angle, the electronic device may determine a deflection direction of the first optical signal based on a deflection direction of the bottom wall of the electronic device, to determine a PPG signal generated by which optical receiver to obtain the heart rate. Therefore, the electronic device can reduce an error due to the deflection angle between the bottom wall of the electronic device and the plane on which the skin of the user is located.

With reference to the third aspect, in a possible implementation, the first accelerometer may be on one side of the straight line. The second accelerometer may be on the other side of the straight line.

With reference to the third aspect, in a possible implementation, the first direction may be a direction perpendicular to the bottom wall. The electronic device may compare the magnitudes of the first acceleration and the second acceleration. If the magnitude of the first acceleration is greater than the magnitude of the second acceleration, the electronic device may select the fourth signal. The electronic device may obtain the heart rate based on the fourth signal. If the magnitude of the second acceleration is greater than the magnitude of the first acceleration, the electronic device may select the second signal. The electronic device may obtain the heart rate based on the second signal.

With reference to the third aspect, in a possible implementation, the first direction may be a direction perpendicular to the bottom wall. The electronic device may compare the magnitudes of the first acceleration and the second acceleration. If the magnitude of the first acceleration is greater than the magnitude of the second acceleration, and a difference between the magnitude of the first acceleration and the magnitude of the second acceleration is greater than a first acceleration difference, the electronic device may select the fourth signal. The electronic device may obtain the heart rate based on the fourth signal. If the magnitude of the second acceleration is greater than the magnitude of the first acceleration, and a difference between the magnitude of the second acceleration and the magnitude of the first acceleration is greater than the first acceleration difference, the electronic device may select the second signal. The electronic device may obtain the heart rate based on the second signal.

With reference to the third aspect, in a possible implementation, the electronic device may select a plurality of signals from the first signal, the second signal, the third signal, and the fourth signal to obtain the heart rate.

Specifically, if the magnitude of the first acceleration is greater than the magnitude of the second acceleration, the electronic device may select the third signal and the fourth signal. The electronic device may add up the third signal and the fourth signal, and obtain the heart rate by using a signal obtained through addition. If the magnitude of the second acceleration is greater than the magnitude of the first acceleration, the electronic device may select the first signal and the second signal. The electronic device may add up the first signal and the second signal, and obtain the heart rate by using a signal obtained through addition.

According to an example not covered by the claims but useful for understanding the application, an embodiment of this application further provides another electronic device. The electronic device includes a bottom wall, at least one first optical transmitter, a first receiver, a second receiver, a third receiver, a fourth receiver, a first accelerometer, and a second accelerometer. The first optical transmitter, the first receiver, the second receiver, the third receiver, and the fourth receiver may be located on one straight line. The first receiver and the second receiver may be distributed on one side of the first optical transmitter. The third receiver and the fourth receiver may be distributed on the other side of the first optical transmitter. A distance between the second receiver and the first optical transmitter may be greater than a distance between the first receiver and the first optical transmitter. A distance between the fourth receiver and the first optical transmitter may be greater than a distance between the third receiver and the first optical transmitter. A spacing between the first accelerometer and the fourth receiver may be less than a first spacing. A spacing between the second accelerometer and the fourth receiver may be less than the first spacing. A first opening and a second opening may be provided on the bottom wall. An optical signal transmitted by the first optical transmitter may be transmitted from the bottom wall through the first opening. The first receiver and the second receiver may receive optical signals through the second opening.

The first optical transmitter may be configured to transmit a first optical signal with a first wavelength. The first wavelength may be greater than or equal to 492 nanometers and less than or equal to 577 nanometers. The first receiver, the second receiver, the third receiver, and the fourth receiver may be configured to receive the first optical signal, and respectively generate a first signal, a second signal, a third signal, and a fourth signal based on the first optical signal.

The first accelerometer may be configured to measure a first acceleration of the electronic device in a first direction. The second accelerometer may be configured to measure a second acceleration of the electronic device in the first direction.

The processor may be configured to compare magnitudes of the first acceleration and the second acceleration. Based on a comparison result, the electronic device may select at least one signal from the first signal, the second signal, the third signal, and the fourth signal, and obtain a heart rate based on the selected at least one signal.

According to a another example not covered by the claims but useful for understanding the application, an embodiment of this application provides a chip, and the chip is applied to an electronic device. The chip includes one or more processors, and the processor is configured to invoke computer instructions to enable the device to execute any one of the possible implementations of the first aspect or the possible implementations of the third aspect.

According to another example not covered by the claims but useful for understanding the application, an embodiment of this application provides a computer program product including instructions. When the computer program product runs on an electronic device, the electronic device is enabled to execute any one of the possible implementations of the first aspect or the possible implementations of the third aspect.

According to another example not covered by the claims but useful for understanding the application, an embodiment of this application provides a computer-readable storage medium, including instructions. When the instructions are run on an electronic device, the electronic device is enabled to execute any one of the possible implementations of the first aspect or the possible implementations of the third aspect.

It may be understood that the electronic device provided in the second aspect, the electronic device provided in the above example, the chip provided in the above example, the computer program product provided in the above example, and the computer-readable storage medium provided in the above example are all used to perform the method provided in embodiments of this application. Therefore, for beneficial effects that can be achieved, refer to the beneficial effects in the corresponding method, and details are not described herein again.

### BRIEF DESCRIPTION OF DRAWINGS

FIG. 1 is a line graph of absorptivity for optical signals absorbed by some human tissues versus wavelengths of the optical signals according to an embodiment of this application;
FIG. 2 is a schematic diagram of a scenario in which an electronic device detects a heart rate by using a PPG according to an embodiment of this application;
FIG. 3 is a schematic diagram of a structure of an electronic device for reducing noise in a PPG signal according to an embodiment of this application;
FIG. 4 is a schematic diagram of a structure of another electronic device for reducing noise in a PPG signal according to an embodiment of this application;
FIG. 5 is a schematic diagram of a structure of another electronic device for reducing noise in a PPG signal according to an embodiment of this application;
FIG. 6A and FIG. 6B are schematic diagrams of a structure of an apparatus for adjusting optical paths of test light and compensation light according to an embodiment of this application;
FIG. 7A and FIG. 7B are schematic diagrams of a structure of another apparatus for adjusting optical paths of test light and compensation light according to an embodiment of this application;
FIG. 8 is a schematic diagram of a structure of another apparatus for adjusting optical paths of test light and compensation light according to an embodiment of this application;
FIG. 9 is a schematic diagram of an application scenario in which deflection occurs between an electronic device and a skin surface of a user according to an embodiment of this application; and
FIG. 10A to FIG. 10F are schematic diagrams of structures of some other electronic devices according to an embodiment of this application.

### DESCRIPTION OF EMBODIMENTS

Terms used in the following embodiments of this application are merely for the purpose of describing specific embodiments, but are not intended to limit this application. The singular expressions "one", "one type of", "the", "the foregoing", and "this" used in this specification and the appended claims of this application are also intended to include plural forms, unless otherwise specified in the context clearly. It should be further understood that the term "and/or" used in this application indicates and includes any one or all of possible combinations of one or more listed items.

In a method for detecting a heart rate by using a PPG by an electronic device, the electronic device includes an optical transmitter and an optical receiver. The electronic device may be worn on a wrist, an abdomen, a leg, or another location of a user, so that the optical transmitter can be close to a skin of the user. The optical transmitter may transmit an optical signal to the skin of the user. The transmitted optical signal may penetrate the skin of the user to a depth of tens of microns to several millimeters. A penetration depth of an optical signal may be determined by parameters of the optical signal, such as a frequency and a wavelength of the optical signal, and a component of a skin layer of a user. In the transmitted optical signal, a part of the optical signal may be absorbed by tissues, such as blood in a blood vessel, a skin, a muscle, and a skeleton of the user, and the other part of the optical signal may be transmitted to the optical receiver through reflecting or scattering or in another manner by the tissues, such as the blood, the skin, the muscle, and the skeleton. The optical receiver receives the optical signal, and may generate a PPG signal. Strength of the PPG signal is associated with strength of the optical signal received by the optical receiver.

The PPG signal may include a direct current (DC for short) signal and an alternating current (AC for short) signal. A volume of the blood in the blood vessel of the user varies with relaxation and contraction of the heart. When the heart relaxes, the blood flows back to the heart through the blood vessel, and the volume of the blood in the blood vessel decreases, so that absorption of the optical signal also decreases. Therefore, the optical receiver can receive a larger part of the optical signal, and the strength of the optical signal is also enhanced. When the heart contracts, the blood flows from the heart to various parts of the body through the blood vessel. The volume of the blood in the blood vessel increases, so that absorption of the optical signal also increases. Therefore, the optical signal received by the optical receiver decreases, and the strength of the optical signal also decreases. Therefore, strength of a part of the PPG signal varies with the relaxation and contraction of the heart. This part of the signal is an AC generated by the optical receiver based on the optical signal reflected or scattered by the blood. A waveform of the AC is usually a periodic sine waveform. The electronic device may calculate a heart rate of the user based on change frequency of signal strength in the AC.

In addition, the skin, the muscle, the skeleton, and another tissue of the user do not vary with the relaxation and contraction of the heart. Absorption of the optical signal by these tissues of the skin, the muscle, and the skeleton is basically unchanged. The optical receiver may generate a DC based on the optical signal reflected or scattered by these tissues of the skin, the muscle, and the skeleton.

In some embodiments, the electronic device may filter out the DC in the PPG signal by using a filter, to obtain the AC in the PPG signal. In this way, the electronic device may calculate the heart rate of the user based on the AC.

The PPG signal used to detect the heart rate may include noise. The noise mainly includes motion noise and random noise.

When the user is in a motion state, both sweat generated by the user and relative movement generated between the electronic device and the skin of the user affect strength of the optical signal received by the optical receiver. Consequently, the PPG signal generated by the optical receiver includes the motion noise. A reason for generating the motion noise is not limited in embodiments of this application.

In addition, a circuit structure in the electronic device may bring random noise to the PPG signal. For example, noise may be added to the PPG signal during transmission or processing of the PPG signal. Alternatively, ambient light may bring random noise to the PPG signal.

The PPG signal including the motion noise and the random noise cannot be used to accurately detect the heart rate.

To reduce the motion noise, by improving tightness of the electronic device worn on the wrist or the leg of the user, a possibility of the relative movement generated between the electronic device and the skin of the user in the motion process may be reduced, to reduce the motion noise. However, this implementation makes the electronic device less comfortable to wear, and may even cause damage to the skin of the user. In addition, whether this implementation can reduce the motion noise is related to a wearing habit of the user, and it is difficult to effectively reduce the motion noise and the random noise and improve accuracy of heart rate detection.

In another solution, the electronic device may detect the motion noise by using a motion sensor, for example, an accelerometer or a gyroscope, to reduce the motion noise in the PPG signal. However, in the method for detecting a heart rate by using a PPG, the optical receiver may receive a plurality of optical signals of different optical paths. The motion noise included in the PPG signal generated by the optical receiver is related to an optical path of the received optical signal. The motion noise detected based on the motion sensor cannot be associated with the optical path of the optical signal received by the optical receiver. In other words, an implementation of reducing the motion noise by using the motion sensor cannot determine a magnitude of the motion noise in the PPG signal. Consequently, it is difficult to effectively reduce the motion noise and the random noise, and improve accuracy of heart rate detection.

Embodiments of this application provide a method for obtaining a heart rate and an electronic device. The electronic device includes at least one test optical transmitter, one compensation optical transmitter, one test optical receiver, and one compensation optical receiver.

The test optical transmitter may be configured to transmit test light. The test light may be an optical signal for which blood has high absorptivity. The optical signal for which blood has high absorptivity is sensitive to a volume change of the blood. To be specific, when a volume of blood in a blood vessel increases, absorbed test light increases significantly. When the volume of blood in the blood vessel decreases, absorbed test light decreases significantly. Therefore, an amplitude of AC change in a PPG signal is large, facilitating accurate heart rate calculation.

The test optical receiver may be configured to receive the test light and generate a test PPG signal.

The compensation optical transmitter may be configured to transmit compensation light. The compensation light may be an optical signal for which blood, a skin, a muscle, a skeleton, and another tissue have low absorptivity. The optical signal for which blood, a skin, a muscle, a skeleton, and another tissue have low absorptivity is insensitive to a volume change of the blood. In other words, regardless of whether the volume of the blood in the blood vessel increases or decreases, absorption of the compensation light by the blood, the skin, the muscle, the skeleton, and the another tissue is basically unchanged or only slightly changed.

The compensation optical receiver may be configured to receive the compensation light, and generate a compensation PPG signal. It can be learned from the characteristic of the compensation light that there is no or only a small quantity of alternating current signals in the compensation PPG signal.

When a user is in a motion state, factors such as sweat generated by the user or relative movement generated between the electronic device and the skin of the user have same or similar impact on the test PPG signal and the compensation PPG signal. In other words, a magnitude of motion noise in the test PPG signal is the same as or similar to a magnitude of motion noise in the compensation PPG signal.

A circuit structure in the electronic device also has same or similar impact on the test PPG signal and the compensation PPG signal. A magnitude of random noise in the test PPG signal is the same as or similar to a magnitude of random noise in the compensation PPG signal.

In this embodiment of this application, the electronic device may subtract the compensation PPG signal from the test PPG signal, and obtain a heart rate of the user by using a PPG signal obtained after subtraction processing. The magnitude of the motion noise in the test PPG signal is the same as or similar to the magnitude of the motion noise in the compensation PPG signal. The magnitude of the random noise in the test PPG signal is the same as or similar to the magnitude of the random noise in the compensation PPG signal. In addition, there is no or only a small quantity of alternating current signals in the compensation PPG signal. The PPG signal obtained after subtraction processing may include an AC used to calculate the heart rate, and basically does not include motion noise and random noise. The foregoing implementations can effectively reduce noise and improve accuracy of heart rate detection.

The following describes a method for selecting the test light and the compensation light.

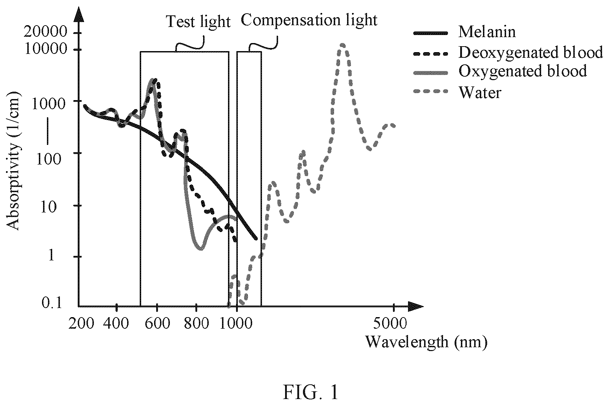

Different tissues of a human body have different absorptivity for optical signals with different wavelengths. FIG. 1 is a line graph of absorptivity for optical signals absorbed by some human tissues versus wavelengths of the optical signals.

As shown in FIG. 1, melanin, deoxygenated blood, oxygenated blood, and water in the human body have different absorptivity for optical signals with different wavelengths. The deoxygenated blood and the oxygenated blood have highest absorptivity for an optical signal with a wavelength of approximately 570 nanometers. That is, the optical signal with a wavelength of approximately 570 nanometers is most sensitive to a volume change of the blood. The optical signal with a wavelength of approximately 570 nanometers may be used as the test light. The test optical transmitter may transmit the optical signal with a wavelength of approximately 570 nanometers. A wavelength of the test light is not limited in this embodiment of this application, and may be any wavelength greater than or equal to 492 nanometers and less than or equal to 577 nanometers.

The melanin, the deoxygenated blood, the oxygenated blood, and the water each have low absorptivity for an optical signal with a wavelength of approximately 1300 nanometers. That is, the optical signal with a wavelength of approximately 1300 nanometers is insensitive to the volume change of the blood. The optical signal with a wavelength of approximately 1300 nanometers may be used as the compensation light. The compensation optical transmitter may transmit the optical signal with a wavelength of approximately 1300 nanometers. A wavelength of the compensation light is not limited in this embodiment of this application, and may be any wavelength greater than or equal to 1000 nanometers and less than or equal to 1500 nanometers.

The following describes a schematic diagram of a scenario in which the electronic device detects the heart rate by using the PPG based on the test light and the compensation light.

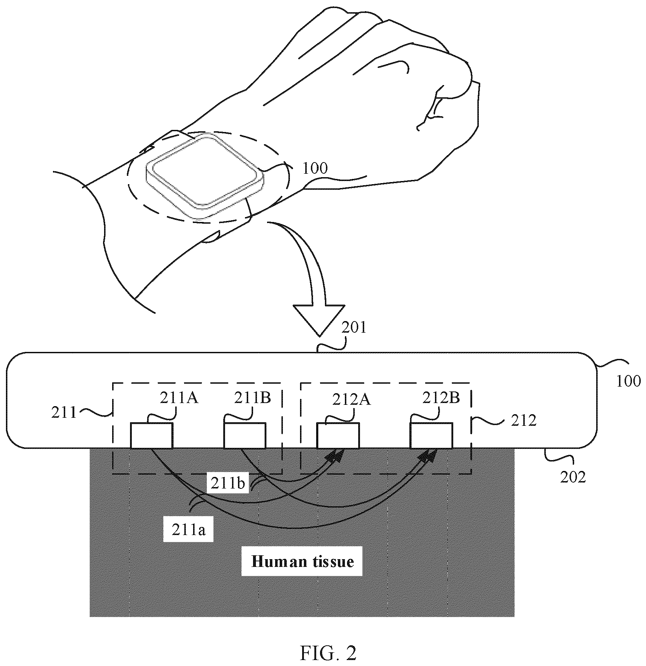

As shown in FIG. 2, an electronic device 100 may be a wearable device, such as a smartwatch. The electronic device 100 may be worn on the wrist of the user. The electronic device 100 may include a bottom wall 202 and an upper surface 201. The bottom wall 202 may be a side close to the skin of the user when the electronic device 100 is worn on the wrist of the user. The bottom wall 202 may include one or more openings. The opening of the bottom wall may be covered by a lens. The electronic device 100 may transmit an optical signal to the skin of the user through the opening, and receive an optical signal reflected or scattered by the human tissue. The upper surface 201 may be a side opposite to the bottom wall 202, for example, a side on which a display screen of the electronic device 100 is located.

The electronic device 100 may include an optical transmitter 211 and an optical receiver 212. The optical transmitter 211 includes at least one test optical transmitter 211A and one compensation optical transmitter 211B. The optical receiver 212 includes at least one test optical receiver 212A and one compensation optical receiver 212B.

The optical transmitter 211 may be, for example, a photon generator of a light emitting diode (light emitting diode, LED), and may be configured to transmit an optical signal with a specific wavelength. The test optical transmitter 211A may be configured to transmit test light 211a, such as an optical signal with a wavelength of 570 nanometers. The compensation optical transmitter 211B may be configured to transmit compensation light 211b, such as an optical signal with a wavelength of 1300 nanometers.

The optical receiver 212 according to claim 7 includes a photoelectric detector and an optical filter. The photoelectric detector may be a photodiode, a phototriode, a photoresistor, a thermistor, or the like. The photoelectric detector may be configured to receive optical signals with different wavelengths, such as an optical signal with any wavelength generated by the photon generator. In response to the received optical signal, the photoelectric detector may generate a PPG signal. The PPG signal may include a current, a voltage, or another electrical parameter. The optical filter may be configured to filter an optical signal with a specific wavelength.

The test optical receiver 212A may receive the test light 211a and/or the compensation light 211b reflected or scattered by the human tissue. The optical filter in the test optical receiver 212A may be configured to filter the compensation light 211b therein, so that the photoelectric detector can generate a test PPG signal related to the test light 211a. The test PPG signal includes an alternating current signal, a direct current signal, motion noise, and random noise. The alternating current signal may be generated based on the received test light 211a reflected or scattered by the blood. The direct current signal may be generated based on the received test light 211a reflected or scattered by the skin, the muscle, the skeleton, and the another tissue. The motion noise may be caused by user motion and is a part of the test PPG signal that affects heart rate detection. The random noise may be caused when the electronic device 100 transmits or processes the test PPG signal.

The compensation optical receiver 212B may receive the test light 211a and/or the compensation light 211b reflected or scattered by the human tissue. The optical filter in the compensation optical receiver 212B may be configured to filter the test light 211a therein, so that the photoelectric detector can generate a compensation PPG signal related to the compensation light 211b. The compensation PPG signal includes a direct current signal, motion noise, and random noise. The direct current signal in the compensation PPG signal may be generated based on the received compensation light 211b reflected or scattered by the skin, the muscle, the skeleton, and the another tissue. A magnitude of the motion noise in the compensation PPG signal is the same as or similar to a magnitude of the motion noise in the test PPG signal. To be specific, for the motion noise in the compensation PPG signal and the motion noise in the test PPG signal that have a same spectrum, amplitudes of the two may be proportional based on a first proportion value. A magnitude of the random noise in the compensation PPG signal is the same as or similar to a magnitude of the random noise in the test PPG signal. To be specific, for the random noise in the compensation PPG signal and the random noise in the test PPG signal that have a same spectrum, amplitudes of the two may be proportional based on a second proportion value. The first proportion value and the second proportion value are not limited in this embodiment of this application.

The electronic device may subtract the compensation PPG signal from the test PPG signal, thereby reducing the motion noise and the random noise in the test PPG signal. The electronic device may calculate motion and health data, such as a heart rate and blood oxygen saturation, based on a PPG signal after subtraction processing.

In this embodiment of this application, locations of the optical transmitter 211 and the optical receiver 212 may correspond to a position of the opening on the bottom wall 202 of the electronic device 100. In other words, the optical transmitter 211 may transmit the optical signal through the opening on the bottom wall 202. The optical receiver 212 may receive the optical signal through the opening on the bottom wall 202. The lens covering the opening may include materials such as glass and a polymer, and may be configured to provide functions such as focusing, collimation, refraction, and diffraction. In addition, the lens may provide mechanical protection for the optical transmitter 211 and the optical receiver 212.

Locations of the test optical transmitter 211A, the compensation optical transmitter 211B, the test optical receiver 212A, and the compensation optical receiver 212B are not limited in this embodiment of this application.

In some embodiments, the locations of the test optical transmitter 211A and the compensation optical transmitter 211B in the electronic device 100 may be close to each other, so that an optical path of the test light 211a transmitted by the test optical transmitter 211A may be the same as or similar to an optical path of the compensation light 211b transmitted by the compensation optical transmitter 211B. Therefore, the user may have same or similar impact on the test light 211a and the compensation light 211b during motion. The test PPG signal generated by the test optical receiver 212A and the compensation PPG signal generated by the compensation optical receiver 212B may include motion noise of a same or similar magnitude. Therefore, keeping the optical path of the test light 211a and the optical path of the compensation light 211b the same or similar can better reduce the motion noise in the test PPG signal, improving accuracy of heart rate detection.

Types of the photon generator, the photoelectric detector, and the optical filter are not limited in this embodiment of this application. For specific manners in which the photon generator transmits an optical signal with a specific wavelength, the photoelectric detector receives the optical signal and generates a PPG signal, and the optical filter filters the optical signal with the specific wavelength, refer to implementations in a conventional technology.

In addition to calculation of the heart rate, the PPG signal obtained by subtracting the compensation PPG signal from the test PPG signal may be further used to calculate other motion and health data, such as blood oxygen saturation. In addition, because the PPG signal obtained after subtraction processing is a PPG signal from which the motion noise and the random noise are removed, the electronic device can improve accuracy of detecting the motion and health data.

In addition to being worn on the wrist of the user, the electronic device 100 may be further worn on an abdomen, a leg, or another location of the user.

The electronic device 100 is not limited in this embodiment of this application, and may be alternatively a smart band, a mobile phone, or another device.

The following describes a schematic diagram of a structure of an electronic device for reducing noise in a PPG signal.

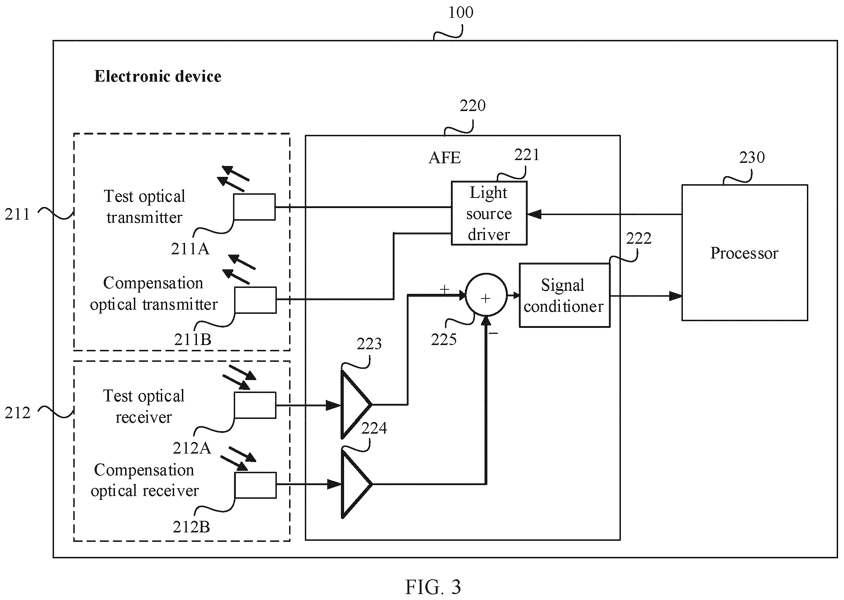

As shown in FIG. 3, an electronic device 100 includes an optical transmitter 211, an optical receiver 212, an analog front end (analog front end, AFE) 220, and a processor 230.

The optical transmitter 211 may include at least one test optical transmitter 211A and one compensation optical transmitter 211B. The optical receiver 212 may include at least one test optical receiver 212A and one compensation optical receiver 212B. For functions of the test optical transmitter 211A, the test optical receiver 212A, the compensation optical transmitter 211B, and the compensation optical receiver 212B, refer to descriptions in the foregoing embodiments. Details are not described herein again.

The AFE 220 may be configured to drive the test optical transmitter 211A and the compensation optical transmitter 211B to transmit optical signals with specific wavelengths, and process PPG signals generated by the test optical receiver 212A and the compensation optical receiver 212B. The AFE 220 may be connected to the test optical transmitter 211A, the test optical receiver 212A, the compensation optical transmitter 211B, the compensation optical receiver 212B, and the processor 230.

The AFE 220 may include a light source driver 221, a signal conditioner 222, a first trans-impedance amplifier (trans-impedance amplifier, TIA) 223, a second TIA 224, and a calculator 225.

The light source driver 221 may be connected to the processor 230, the test optical transmitter 211A, and the compensation optical transmitter 211B. The light source driver 221 may be configured to receive instructions from the processor 230, to drive the test optical transmitter 211A to transmit test light (for example, an optical signal with a wavelength of 570 nanometers) and drive the compensation optical transmitter 211B to transmit compensation light (for example, an optical signal with a wavelength of 1300 nanometers).

The first TIA 223 and the second TIA 224 may be configured to convert current signals into voltage signals and amplify the voltage signals. The test optical receiver 212A may generate a first test PPG signal whose electrical parameter is a current based on the test light reflected or scattered by a human tissue. The test optical receiver 212A may be connected to the first TIA 223. The first TIA 223 may convert the test PPG signal whose electrical parameter is a current into a second test PPG signal whose electrical parameter is a voltage, and amplify the second test PPG signal. A gain of the first TIA 223 to the second test PPG signal may be m. The compensation optical receiver 212B may generate a first compensation PPG signal whose electrical parameter is a current based on the compensation light reflected or scattered by the human tissue. The compensation optical receiver 212B may be connected to the second TIA 224. The second TIA 224 may convert the first compensation PPG signal whose electrical parameter is a current into a second compensation PPG signal whose electrical parameter is a voltage, and amplify the second compensation PPG signal. A gain of the second TIA 224 to the second compensation PPG signal may be n. Both m and n are positive numbers.

In this embodiment of this application, a magnification factor m of the first TIA 223 and a magnification factor n of the second TIA 224 may be the same or different.

The calculator 225 may be configured to perform arithmetic and logical operations on a signal, such as adding, subtracting, multiplying, and dividing. Specifically, the calculator 225 may be connected to the first TIA 223 and the second TIA 224. The calculator 225 may subtract the second compensation PPG signal of the second TIA 224 from the second test PPG signal of the first TIA 223, and send a PPG signal obtained after subtraction processing to the signal conditioner 222.

The signal conditioner 222 may include one or more of the following components: an amplifier, a filter, a sampler, and an analog-to-digital converter. The signal conditioner 222 may be connected to the calculator 225, and may be configured to perform one or more of the following processing on a PPG signal obtained through processing by the calculator 225: amplification, filtering, sampling, and analog-to-digital conversion. The signal conditioner 222 may be connected to the processor 230, to send a PPG signal obtained through processing by the signal conditioner 222 to the processor 230.

The processor 230 may be connected to the AFE 220. Specifically, the processor 230 may be connected to the light source driver 221 and the signal conditioner 222 in the AFE 220. In response to a user operation instructing the electronic device to perform heart rate detection, the processor 230 may send an instruction to the light source driver 221. The instruction is used to instruct the light source driver 221 to drive the test optical transmitter 211A and the compensation optical transmitter 211B to transmit optical signals. Therefore, the test optical transmitter 211A and the compensation optical transmitter 211B can simultaneously or time-divisionally transmit optical signals with specific wavelengths. When receiving a PPG signal from the signal conditioner 222, the processor 230 may calculate a heart rate based on the PPG signal.

In some embodiments, the electronic device 100 may include a plurality of test optical transmitters. The plurality of test optical transmitters may be configured to transmit test light, such as optical signals with wavelengths in a range greater than or equal to 492 nanometers and less than or equal to 577 nanometers. The plurality of test optical transmitters may transmit optical signals with a same wavelength or different wavelengths.

In some embodiments, the electronic device 100 may include a plurality of compensation optical transmitters. The plurality of compensation optical transmitters may be configured to transmit compensation light, such as optical signals with wavelengths in a range greater than or equal to 1000 nanometers and less than or equal to 1500 nanometers. The plurality of compensation optical transmitters may transmit optical signals with a same wavelength or different wavelengths.

A module in the electronic device 100 is not limited in this embodiment of this application, and the electronic device 100 may further include more or fewer modules.

Based on the schematic diagram of the structure of the electronic device shown in FIG. 3, the following describes a principle of reducing noise in the PPG signal.

The test optical receiver 212A may generate the first test PPG signal whose electrical parameter is a current. The first TIA 223 may convert the first test PPG signal into the second test PPG signal Xₜ whose electrical parameter is a voltage. The second test PPG signal Xt may include a direct current signal X_{t-dc}, an alternating current signal X_{t-ac}, motion noise Xₜ₋ᵢₙₜ, and random noise Xₜ₋ₙₒᵢₛₑ. A gain of the first TIA 223 to the second test PPG signal may be m.

The compensation optical receiver 212B may generate the first compensation PPG signal whose electrical parameter is a current. The second TIA 224 may convert the first compensation PPG signal into the second compensation PPG signal X_{c} whose electrical parameter is a voltage. The second compensation PPG signal X_{c} may include a direct current signal X_{c-dc}, an alternating current signal X_{c-ac}, motion noise X_{c-int}, and random noise X_{c-noise.} A gain of the second TIA 224 to the second compensation PPG signal may be n.

The calculator 225 may perform subtraction processing on the second test PPG signal and the second compensation PPG signal, to obtain a PPG signal Y after subtraction processing. Herein, Y = mXt - nX_{c}. That is, Y = m(X_{t-dc} + X_{t-ac} + Xₜ₋ᵢₙₜ + Xₜ₋ₙₒᵢₛₑ) - n(X_{c-dc} + X_{c-ac} + X_{c-int} + X_{c-noise}). In this case, the following formulas are obtained: ${Y}_{dc} = {mX}_{t - dc} - {nX}_{c - dc} ;$ ${Y}_{ac} = {mX}_{t - ac} - {nX}_{c - ac} ;$ ${Y}_{int} = {mX}_{t - int} - {nX}_{c - int} ;$ and ${Y}_{noise} = sqrt \left({\left({mX}_{t - noise}\right)}^{2}+{\left({nX}_{c - noise}\right)}^{2}\right) ,$ where sqrt represents square root calculation.

The first TIA 223 and the second TIA 224 in the electronic device 100 may adjust the gain of the signal, so that n = mX_{t-dc}/X_{c-dc}. Therefore, a direct current signal in the PPG signal Y obtained through subtraction processing by the calculator 225 may approach zero.

Because the compensation light is insensitive to a volume change of blood in a blood vessel, and an amount of the compensation light absorbed by the blood is basically unchanged in a process of increasing or decreasing a volume of the blood, the alternating current signal X_{c-ac} in the second compensation PPG signal approaches zero. Strength of an alternating current signal Y_{ac} in the PPG signal Y is the same as or similar to strength of the alternating current signal mX_{t-ac} in the second test PPG signal amplified by the first TIA 223.

When a user is in a motion state, factors such as sweat generated by the user or relative movement generated between the electronic device and a skin of the user have same or similar impact on the second test PPG signal and the second compensation PPG signal. Magnitudes of the motion noise Xₜ₋ᵢₙₜ in the second test PPG signal and the motion noise X_{c-int} in the second compensation PPG signal are the same or similar. The motion noise in the PPG signal Y is much smaller than the motion noise mXₜ₋ᵢₙₜ in the second test PPG signal amplified by the first TIA 223.

It can be learned from the foregoing description that, without consideration of the random noise, a signal-to-noise ratio Y_{ac}/Yᵢₙₜ of the PPG signal Y obtained through subtraction processing by the calculator 225 is much higher than a signal-to-noise ratio X_{t-ac}/Xₜ₋ᵢₙₜ of the second test PPG signal Xt. The motion noise in the PPG signal Y is greatly reduced compared with that in the second test PPG signal. The processor in the electronic device 100 calculates the heart rate based on the alternating current signal Y_{ac} in the PPG signal Y. This can effectively improve accuracy of heart rate detection.

In some embodiments, the electronic device 100 further includes a motion sensor (for example, an accelerometer or a gyroscope). The electronic device 100 may detect, by using the motion sensor, whether the user is in the motion state. If it is determined that the user is not in the motion state, the light source driver 221 in the AFE 220 may drive the test optical transmitter 211A to transmit the test light without driving the compensation optical transmitter 211B to transmit the optical signal. In addition, the test optical receiver 212A may be in a working state, and the compensation optical receiver 212B may be in a sleep state. When the user is not in the motion state, the motion noise approaches zero. Therefore, when calculating the heart rate, the electronic device 100 may directly perform calculation by using the test PPG signal generated by the test optical receiver 212A. This helps reduce power consumption of the electronic device 100.

In some embodiments, the electronic device 100 may include only one optical receiver. The optical receiver may be configured to receive the test light or the compensation light reflected or scattered by the human tissue. The electronic device 100 may time-divisionally transmit test light and compensation light for a plurality of times. For example, within a period of time in which the electronic device 100 transmits an optical signal to detect a heart rate, a plurality of first time periods and a plurality of second time periods may be included. An interval between the first time period and the second time period may be less than first duration. A magnitude of the first duration may approach zero, and the magnitude of the first duration is not limited in this embodiment of this application.

In the first time period, the light source driver 221 may drive the test optical transmitter 211A to transmit test light. The optical receiver may receive the test light reflected or scattered by the human tissue and generate a test PPG signal. In the second time period, the light source driver 221 may drive the compensation optical transmitter 211B to transmit compensation light. The optical receiver may receive the compensation light reflected or scattered by human tissue, and generate a compensation PPG signal. Further, the electronic device 100 may subtract the compensation PPG signal from the test PPG signal, and calculate the heart rate by using a PPG signal after subtraction processing.

In some embodiments, the electronic device 100 may include a plurality of optical transmitters transmitting different wavelengths. The plurality of optical transmitters may include: a green light transmitter that transmits an optical signal whose wavelength is within a wavelength range (for example, 570 nanometers) of green light, a red light transmitter that transmits an optical signal whose wavelength is within a wavelength range (for example, 670 nanometers) of red light, and an infrared light transmitter that transmits an optical signal whose wavelength is within a wavelength range (for example, 940 nanometers) of infrared light.

The optical receiver of the electronic device 100 may generate a green PPG signal X_{g} based on received green light. The green PPG signal may include a direct current signal X_{g-dc}, an alternating current signal X_{g-ac}, motion noise X_{g-int}, and random noise X_{g-noise}. That is, X_{g} = X_{g-dc} + X_{g-ac} + X_{g-int} + X_{g-noise}.

The optical receiver of the electronic device 100 may generate a red PPG signal Xᵣ based on received red light. The red PPG signal may include a direct current signal X_{r-dc}, an alternating current signal X_{r-ac}, motion noise Xᵣ₋ᵢₙₜ, and random noise Xᵣ₋ₙₒᵢₛₑ. That is, Xᵣ = X_{r-dc} + X_{r-ac} + Xᵣ₋ᵢₙₜ + Xᵣ₋ₙₒᵢₛₑ.

The optical receiver of the electronic device 100 may generate an infrared PPG signal Xᵢᵣ based on received infrared light. The infrared PPG signal may include a direct current signal X_{ir-dc}, an alternating current signal X_{ir-ac}, motion noise Xᵢᵣ₋ᵢₙₜ, and random noise Xᵢᵣ₋ₙₒᵢₛₑ. That is, Xᵢᵣ = X_{ir-dc} + X_{ir-ac} + Xᵢᵣ₋ᵢₙₜ + Xᵢᵣ₋ₙₒᵢₛₑ.

The electronic device can reduce noise (such as the motion noise and the random noise) in the red PPG signal and the infrared PPG signal, and calculate blood oxygen saturation by using a noise-reduced red PPG signal and a noise-reduced infrared PPG signal. The blood oxygen saturation may be calculated according to the following formula (1): $Blood oxygen saturation = A ∗ \frac{{X}_{r - ac} / {X}_{r - dc}}{{X}_{ir - ac} / {X}_{ir - dc}} + B$

In the formula, A and B are system parameters, and specific values of A and B are not limited in this embodiment of this application.

From the line graph of absorptivity for optical signals absorbed by some human tissues versus wavelengths of the optical signals shown in FIG. 1, it can be learned that the blood has low absorptivity for both an optical signal in the wavelength range of red light and an optical signal in the wavelength range of infrared light. In other words, the red light and the infrared light are insensitive to the volume change of the blood in the blood vessel. Both the alternating current signal X_{r-ac} in the red PPG signal and the alternating current signal X_{ir-ac} in the infrared PPG signal are small in magnitude. A difference between a magnitude of the motion noise Xᵣ₋ᵢₙₜ in the red PPG signal and a magnitude of the alternating current signal X_{r-ac} is small, or a magnitude of the motion noise Xᵣ₋ᵢₙₜ is far greater than a magnitude of the alternating current signal X_{r-ac}. A difference between a magnitude of the motion noise Xᵢᵣ₋ᵢₙₜ in the infrared PPG signal and a magnitude of the alternating current signal X_{ir-ac} is small, or a magnitude of the motion noise Xᵢᵣ₋ᵢₙₜ is far greater than a magnitude of the alternating current signal X_{ir-ac}. It is difficult for the electronic device 100 to remove the motion noise from the red PPG signal and the infrared PPG signal.

However, the blood has high absorptivity for an optical signal in the wavelength range of green light. In other words, the green light is sensitive to the volume change of the blood in the blood vessel. The alternating current signal X_{g-ac} in the green PPG signal is large in magnitude. A magnitude of the alternating current signal X_{g-ac} in the green PPG signal is generally greater than those of the motion noise X_{g-int} and the random noise X_{g-noise}. The electronic device 100 can easily separate the motion noise and the random noise from the green PPG signal, where X_{g-int} + X_{g-noise} = X_{g} - X_{g-dc} - X_{g-ac}. For a method for separating the motion noise and the random noise from the green PPG signal by the electronic device 100, refer to the method in the embodiments shown in FIG. 3, FIG. 4, and FIG. 5. Details are not described herein again.

When the user is in the motion state, the factors such as sweat generated by the user or relative movement generated between the electronic device and a skin of the user have same or similar impact on the green PPG signal, the red PPG signal, and the infrared PPG signal. Magnitudes of the motion noise in the green PPG signal, the motion noise in the red PPG signal, and the motion noise in the infrared PPG signal are the same or similar.

The electronic device 100 can reduce noise in the red PPG signal and noise in the infrared PPG signal by using the noise (such as the motion noise and the random noise) separated from the green PPG signal. Specifically, a noise-reduced red PPG signal may be ${X}_{r}^{′}$, and a noise-reduced infrared PPG signal may be ${X}_{ir}^{′}$, where ${X}_{r}^{′} = {X}_{r} - k ∗ \left({X}_{g - int}+{X}_{g - noise}\right) =$ and ${X}_{ir}^{′} = {X}_{ir} - k ∗ \left({X}_{g - int}+{X}_{g - noise}\right)$. Herein, k is a positive number.

It can be learned from the foregoing analysis that the electronic device 100 can reduce the noise in the red PPG signal and the noise in the infrared PPG signal. The noise-reduced red PPG signal and the noise-reduced infrared PPG signal are used to calculate the blood oxygen saturation. The electronic device can improve accuracy of calculating the blood oxygen saturation.

The following describes a schematic diagram of a structure of another electronic device for reducing noise in a PPG signal.

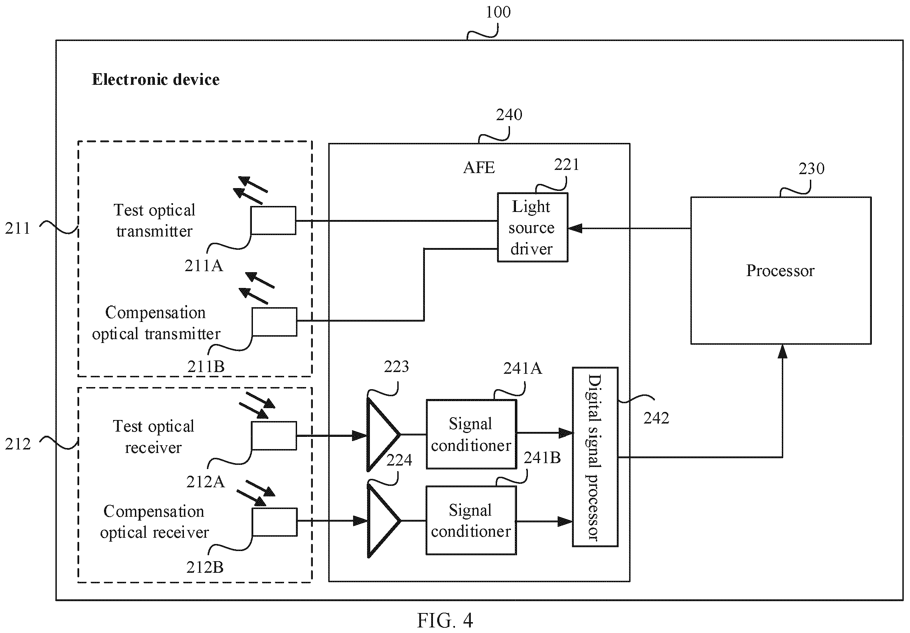

As shown in FIG. 4, an electronic device 100 includes an optical transmitter 211, an optical receiver 212, an AFE 240, and a processor 230. Specifically:
For functions of the optical transmitter 211 and the optical receiver 212, refer to descriptions in the foregoing embodiments. Details are not described herein again.

Like the electronic device 100 shown in FIG. 3, the optical transmitter 211 may include at least one test optical transmitter 211A and one compensation optical transmitter 211B. The optical receiver 212 may include at least one test optical receiver 212A and one compensation optical receiver 212B.

The AFE 240 may be configured to drive the test optical transmitter 211A and the compensation optical transmitter 211B to transmit optical signals with specific wavelengths, and process PPG signals generated by the test optical receiver 212A and the compensation optical receiver 212B. The AFE 240 may be connected to the test optical transmitter 211A, the test optical receiver 212A, the compensation optical transmitter 211B, the compensation optical receiver 212B, and the processor 230.

The AFE 240 may include a light source driver 221, a first TIA 223, a second TIA 224, a signal conditioner 241A, a signal conditioner 241B, and a digital signal processor 242.

For functions of the light source driver 221, the first TIA 223, the second TIA 224, the signal conditioner 241A, and the signal conditioner 241B, refer to the foregoing descriptions of the electronic structure shown in FIG. 3. Details are not described herein again.

It should be noted that when a first test PPG signal is received from the test optical receiver 212A, the first TIA 223 may convert the first test PPG signal whose electrical parameter is a current into a second test PPG signal whose electrical parameter is a voltage, and amplify the second test PPG signal. A gain of the first TIA 223 for amplifying the second test PPG signal may be m. Then, the first TIA 223 may send an amplified second test PPG signal to the signal conditioner 241A. The signal conditioner 241A may perform analog-to-digital conversion on the amplified second test PPG signal, and convert the second test PPG signal that is an analog signal into a third test PPG signal that is a digital signal. The signal conditioner 241A may further perform one or more of the following processing on the amplified second test PPG signal: amplification, filtering, and sampling.

When receiving a first compensation PPG signal from the compensation optical receiver 212B, the second TIA 224 may convert the first compensation PPG signal whose electrical parameter is a current into a second compensation PPG signal whose electrical parameter is a voltage, and amplify the second compensation PPG signal. A gain of the second TIA 224 for amplifying the second compensation PPG signal may be n. Then, the second TIA 224 may send an amplified second compensation PPG signal to the signal conditioner 241B. The signal conditioner 241B may perform analog-to-digital conversion on the amplified second compensation PPG signal, and convert the second compensation PPG signal that is an analog signal into a third compensation PPG signal that is a digital signal. The signal conditioner 241B may further perform one or more of the following processing on the amplified second compensation PPG signal: amplification, filtering, and sampling.

Further, the signal conditioner 241A may send the third test PPG signal that is a digital signal to the digital signal processor 242. The signal conditioner 241B may send the third compensation PPG signal that is a digital signal to the digital signal processor 242. The digital signal processor 242 can reduce motion noise and random noise that are in the third test PPG signal by using the third compensation PPG signal. To be specific, the digital signal processor 242 may subtract the third compensation PPG signal from the third test PPG signal, and send a PPG signal obtained after subtraction processing to the processor 230, to detect a heart rate.

In the electronic device 100 shown in FIG. 4, the electronic device 100 may convert the PPG signal into the digital signal, to reduce the motion noise in the PPG signal in a digital domain.

The processor 230 may be connected to the AFE 240. Specifically, the processor 230 may be connected to the light source driver 221 and the digital signal processor 242 in the AFE 240. In response to a user operation instructing the electronic device to perform heart rate detection, the processor 230 may send an instruction to the light source driver 221. The instruction is used to instruct the light source driver 221 to drive the test optical transmitter 211A and the compensation optical transmitter 211B to transmit optical signals. Therefore, the test optical transmitter 211A and the compensation optical transmitter 211B can simultaneously or time-divisionally transmit optical signals with specific wavelengths. When receiving a PPG signal from the digital signal processor 242, the processor 230 may calculate the heart rate based on the PPG signal.

A module in the electronic device 100 shown in FIG. 4 is not limited in this embodiment of this application, and the electronic device 100 may further include more or fewer modules.

A principle of reducing the noise in the PPG signal by the electronic device 100 shown in FIG. 4 is consistent with a principle of reducing the noise in the PPG signal by the electronic device 100 shown in FIG. 3. That is, in the two principles, the compensation PPG signal is used to reduce the motion noise and the random noise in the test PPG signal. The electronic device 100 may calculate the heart rate based on the PPG signal obtained by subtracting the compensation PPG signal from the test PPG signal. This can effectively improve accuracy of heart rate detection.

The following describes a schematic diagram of a structure of another electronic device for reducing noise in a PPG signal.

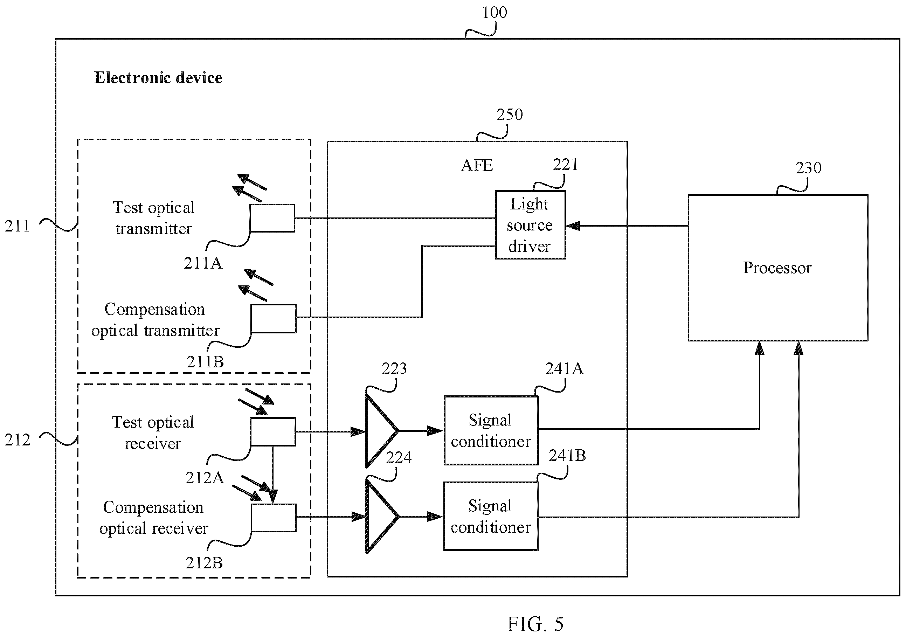

As shown in FIG. 5, an electronic device 100 includes an optical transmitter 211, an optical receiver 212, an AFE 250, and a processor 230.

For functions of the optical transmitter 211 and the optical receiver 212, refer to descriptions in the foregoing embodiments. Details are not described herein again.

Like the electronic device 100 shown in FIG. 3, the optical transmitter 211 may include at least one test optical transmitter 211A and one compensation optical transmitter 211B. The optical receiver 212 may include at least one compensation optical transmitter 211B and one compensation optical receiver 212B.

Unlike the electronic device 100 shown in FIG. 3, in the electronic device shown in FIG. 5, the test optical receiver 212A and the compensation optical receiver 212B may be connected in series. The compensation optical receiver 212B may perform offloading for the test optical receiver 212A. Specifically, the test optical receiver 212A may generate, based on received test light, a test PPG signal whose electrical parameter is a current signal. The compensation optical receiver 212B may generate, based on received compensation light, a compensation PPG signal whose electrical parameter is a current signal. Because the test optical receiver 212A and the compensation optical receiver 212B are connected in series, some current signals in the test PPG signal generated by the test optical receiver 212A can flow to the compensation optical receiver 212B. A magnitude of the current signal flowing to the compensation optical receiver 212B may be the same as or similar to a magnitude of the compensation PPG signal.

It can be learned from the foregoing characteristic of the compensation light that a human tissue (for example, blood, a skin, a muscle, or a skeleton) has low absorptivity for the compensation light, and the compensation PPG signal includes no or only a small quantity of alternating current signals, and includes noise (such as motion noise and random noise). A magnitude of the noise in the compensation PPG signal is the same as or similar to a magnitude of noise in the test PPG signal.

Therefore, a test PPG signal output by the test optical receiver 212A to a first TIA 223 is equivalent to a signal in which motion noise and random noise are reduced. A compensation PPG signal output by the compensation optical receiver 212B to a second TIA 224 is equivalent to a signal in which motion noise and random noise are added.

The AFE 250 may be configured to drive the test optical transmitter 211A and the compensation optical transmitter 211B to transmit optical signals with specific wavelengths, and process PPG signals generated by the test optical receiver 212A and the compensation optical receiver 212B. The AFE 250 may be connected to the test optical transmitter 211A, the test optical receiver 212A, the compensation optical transmitter 211B, the compensation optical receiver 212B, and the processor 230.

The AFE 250 may include a light source driver 221, the first TIA 223, the second TIA 224, a signal conditioner 241A, and a signal conditioner 241B. For functions of the light source driver 221, the first TIA 223, the second TIA 224, the signal conditioner 241A, and the signal conditioner 241B, refer to descriptions in the foregoing embodiments. Details are not described herein again.

The test optical receiver 212A may output a test PPG signal in which motion noise and random noise are reduced, and the test PPG signal may be successively processed by the first TIA 223 and the signal conditioner 241A, and then input to the processor 230. The processor 230 may calculate motion and health data such as a heart rate and blood oxygen saturation by using a test PPG signal from the signal conditioner 241A. The compensation optical receiver 212B may output a compensation PPG signal including motion noise and random noise, and the compensation PPG signal may be successively processed by the second TIA 224 and the signal conditioner 241B, and then output to the processor 230. The compensation PPG signal may be used by the processor 230 to determine a motion state of a user.

A method for using the compensation PPG signal by the processor 230 is not limited in this embodiment of this application.

The test optical receiver 212A and the compensation optical receiver 212B are connected in series. When the test optical receiver 212A generates the test PPG signal, the motion noise and the random noise in the test PPG signal can be reduced. Therefore, the AFE may not need to be equipped with a calculator or a digital signal processor to perform subtraction processing on the test PPG signal and the compensation PPG signal. Therefore, a circuit structure in the AFE can be simplified, and a speed at which the electronic device calculates the heart rate can be improved.

A principle of reducing the noise in the PPG signal by the electronic device 100 shown in FIG. 5 is consistent with a principle of reducing the noise in the PPG signal by the electronic device 100 shown in FIG. 3. That is, in the two principles, the compensation PPG signal is used to reduce the motion noise and the random noise in the test PPG signal. The electronic device 100 may calculate the heart rate based on the PPG signal obtained by subtracting the compensation PPG signal from the test PPG signal. This can effectively improve accuracy of heart rate detection.

In the foregoing electronic devices for reducing the noise in the PPG signal shown in FIG. 3, FIG. 4, and FIG. 5, an optical path of the test light transmitted by the electronic device is the same as or similar to an optical path of the compensation light, and optical path parameters (such as a spot size of an optical signal transmitted to a location or optical signal strength distribution of a spot) are also the same or similar. In other words, the test light and the compensation light can pass through the human tissue along the same or similar paths. Therefore, impact of the user motion on absorption, reflection, scattering, and the like of the test light and the compensation light that are in a same optical path may be the same or similar, so that magnitudes of the motion noise in the test PPG signal and the compensation PPG signal may be the same or similar. The electronic device can more effectively reduce the motion noise in the test PPG signal by using the compensation PPG signal, improving accuracy of heart rate detection.

The following describes a schematic diagram of a structure of an apparatus in an electronic device for adjusting optical paths of test light and compensation light.

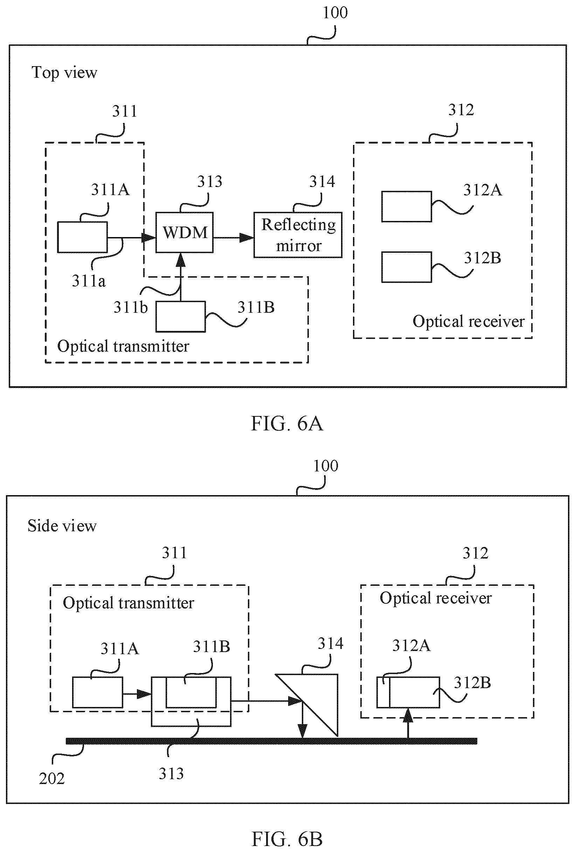

FIG. 6A and FIG. 6B are respectively examples of a top view and a side view of an electronic device 100.

As shown in FIG. 6A, an electronic device 100 may include an optical transmitter 311, an optical receiver 312, a wavelength division multiplexer (wavelength division multiplexing, WDM) 313, and a reflecting mirror 314.

The optical transmitter 311 may include at least two optical transmitters: a first optical transmitter 311A and a second optical transmitter 311B. The first optical transmitter 311A may transmit a first optical signal 311a to the WDM 313. The first optical signal 311a may be the test light mentioned in the foregoing embodiments, for example, the optical signal with a wavelength in a range greater than or equal to 492 nanometers and less than or equal to 577 nanometers. The second optical transmitter 311B may transmit a second optical signal 311b to the WDM 313. The second optical signal 311b may be the compensation light mentioned in the foregoing embodiments, for example, the optical signal with a wavelength in a range greater than or equal to 1000 nanometers and less than or equal to 1500 nanometers.

Optionally, the first optical signal 311a may be the foregoing compensation light, and the second optical signal 311b may be the foregoing test light. Specific locations of the first optical transmitter 311A and the second optical transmitter 311B are not limited in this embodiment of this application.

The WDM 313 may be configured to combine optical signals with different wavelengths into one beam, and transmit the plurality of optical signals with different wavelengths along a same optical path. The first optical transmitter 311A and the second optical transmitter 311B may separately transmit optical signals to the WDM 313. When receiving the test light and the compensation light, the WDM 313 may combine the two optical signals with different wavelengths into one beam, and transmit the beam in a direction in which the reflecting mirror 314 is located.

As shown in FIG. 6B, the reflecting mirror 314 may be configured to reflect test light and compensation light that have a same optical path and that come from the WDM 313. The test light and the compensation light reflected by the reflecting mirror 314 may be transmitted to a skin surface of a user through an opening on a bottom wall 202 of the electronic device.

The optical receiver 312 may include at least two optical receivers: a first optical receiver 312A and a second optical receiver 312B. The first optical receiver 312A may be configured to receive the first optical signal 311a, and generate a first PPG signal related to the first optical signal 311a. The second optical receiver 312B may be configured to receive the second optical signal 311b, and generate a second PPG signal related to the second optical signal 311b.

For example, the first optical receiver 312A may be the test optical receiver mentioned in the foregoing embodiments, and may be configured to receive test light reflected or scattered by a human tissue, and generate a test PPG signal related to the test light. The first optical receiver 312A may further receive compensation light reflected or scattered by the human tissue. An optical filter included in the first optical receiver 312A may filter the received compensation light. The second optical receiver 312B may be the compensation optical receiver mentioned in the foregoing embodiments, and may be configured to receive compensation light reflected or scattered by the human tissue, and generate a compensation PPG signal related to the compensation light. The second optical receiver 312B may further receive test light reflected or scattered by the human tissue. An optical filter included in the second optical receiver 312B may filter the received test light.

Optionally, the first optical receiver 312A may be the foregoing compensation optical receiver, and the second optical receiver 312B may be the foregoing test optical receiver.

In some embodiments, the first optical receiver 312A and the second optical receiver 312B may be distributed symmetrically with respect to an axis formed by a light exit point on the reflecting mirror and the WDM 313.

Locations of the first optical receiver 312A and the second optical receiver 312B are not limited in this embodiment of this application.

It can be learned from the foregoing embodiments that through the WDM 313 and the reflecting mirror 314, the test light and the compensation light may be transmitted along a same optical path to the skin surface of the user. Therefore, the electronic device 100 may enable the user during motion to have same or similar impact on absorption, reflection, and scattering of the test light and the compensation light, so that a magnitude of motion noise in the test PPG signal and a magnitude of motion noise in the compensation PPG signal are the same or similar. The electronic device 100 can more effectively reduce the motion noise in the test PPG signal by using the compensation PPG signal, improving accuracy of heart rate detection.

In some embodiments, the optical receiver 312 in the electronic device 100 may include only one optical receiver. The optical receiver may be configured to receive the test light or the compensation light reflected or scattered by the human tissue. The electronic device 100 may time-divisionally transmit the test light and the compensation light for a plurality of times. For example, within a period of time in which the electronic device 100 transmits an optical signal to detect a heart rate, a plurality of first time periods and a plurality of second time periods may be included. Preferably, the first time period and the second time period may be adjacent time periods. Alternatively, the first time period and the second time period may be non-adjacent time periods. In the first time period, the first optical transmitter 311A may transmit the first optical signal 311a. The optical receiver may receive the first optical signal 311a reflected or scattered by the human tissue, and generate the first PPG signal. In the second time period, the second optical transmitter 311B may transmit the second optical signal 311b. The optical receiver may receive the second optical signal 311b reflected or scattered by the human tissue, and generate the second PPG signal. One of the first PPG signal and the second PPG signal is the foregoing test PPG signal, and the other is the foregoing compensation PPG signal. The electronic device 100 may perform subtraction processing by using the first PPG signal and the second PPG signal, to reduce the motion noise and random noise in the PPG signal, and calculate the heart rate by using a PPG signal obtained after subtraction processing.

The following describes a schematic diagram of a structure of another apparatus for adjusting optical paths of test light and compensation light.

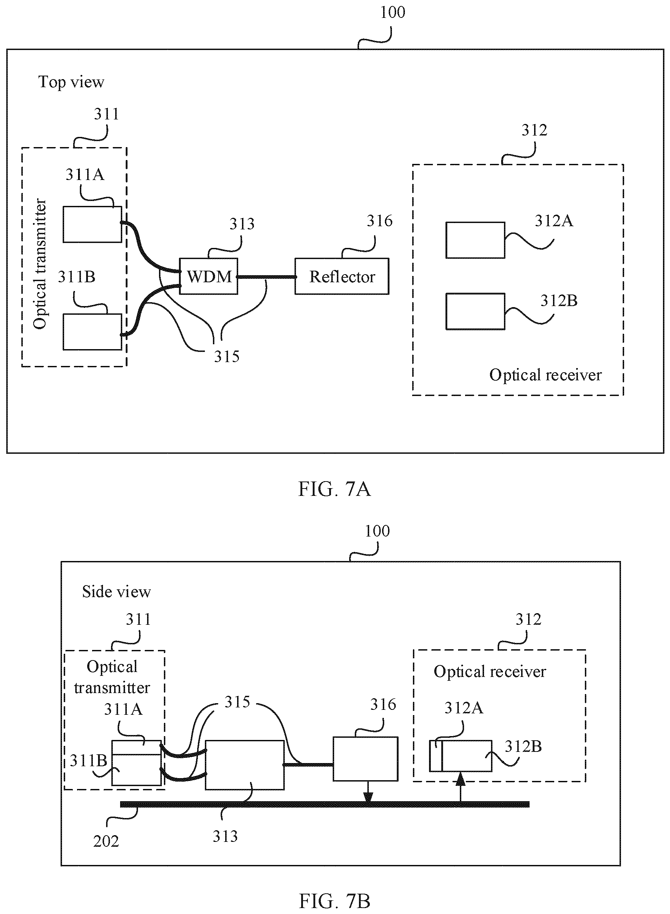

FIG. 7A and FIG. 7B are respectively examples of a top view and a side view of an electronic device 100.

As shown in FIG. 7A, an electronic device 100 may include an optical transmitter 311, an optical receiver 312, a WDM 313, a waveguide 315, and a reflector 316. Specifically:
The optical transmitter 311 may include at least two optical transmitters: a first optical transmitter 311A and a second optical transmitter 311B. The optical receiver 312 may include at least two optical receivers: a first optical receiver 312A and a second optical receiver 312B. For functions of the first optical transmitter 311A, the second optical transmitter 311B, the first optical receiver 312A, and the second optical receiver 312B, refer to the foregoing descriptions of the electronic device shown in FIG. 6A. Details are not described herein again.

For a function of the WDM 313, refer to the foregoing description of the electronic device shown in FIG. 6A. Details are not described herein again.

The waveguide 315 may be configured to receive and transmit optical signals transmitted by the first optical transmitter 311A and the second optical transmitter 311B. The waveguide 315 may transmit, to the WDM 313, the optical signals transmitted by the first optical transmitter 311A and the second optical transmitter 311B. In other words, a first optical signal 311a transmitted by the first optical transmitter 311A and a second optical signal 311b transmitted by the second optical transmitter may be transmitted to the WDM 313 along two waveguides, respectively.

The WDM 313 may combine the two optical signals with different wavelengths into one beam, and transmit the beam to the reflector 316. An optical signal transmitted after the WDM 313 performs multiplexing may be received by the waveguide 315, and transmitted to the reflector 316. In other words, the first optical signal 311a and the second optical signal 311b have a same optical path, and may be transmitted to the reflector 316 along a same waveguide.

As shown in FIG. 7B, the reflector 316 may be configured to reflect test light and compensation light that have a same optical path and that come from the WDM 313. The test light and the compensation light reflected by the reflector 316 may be transmitted to a skin surface of a user through an opening on a bottom wall 212 of the electronic device. The reflector 316 may be the reflecting mirror 314 shown in FIG. 6B. The reflector 316 may be alternatively a grating, for example, a fiber Bragg grating or a vertically coupled grating. The grating may be configured to reflect an optical signal.

The electronic device 100 shown in FIG. 7A and FIG. 7B transmits the optical signal by using the waveguide. This can reduce a loss in a process in which the optical signal is transmitted from the optical transmitter to the WDM and then sent to the reflector. Therefore, the electronic device can improve consistency of optical path parameters of the test light and the compensation light, to more effectively reduce motion noise in a PPG signal, improving accuracy of heart rate detection.

In the foregoing schematic diagram of a structure of an apparatus for adjusting optical paths of test light and compensation light shown in FIG. 6A and FIG. 7A, the optical transmitter 311 may include a plurality of optical transmitters.

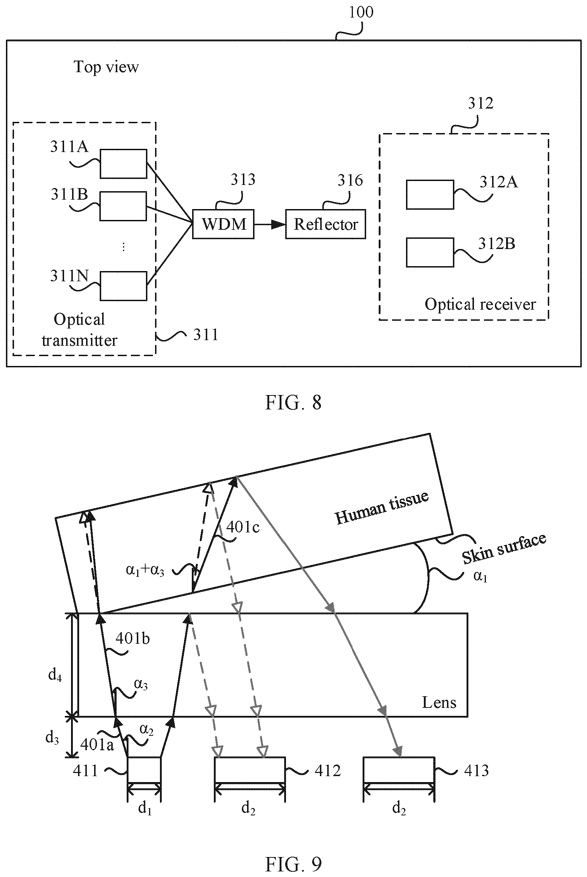

As shown in FIG. 8, the optical transmitter 311 may include a first optical transmitter 311A, a second optical transmitter 311B, ..., and an N^{th} optical transmitter 311N. Herein, N is an integer greater than 2. The plurality of optical transmitters may be configured to transmit optical signals with different wavelengths. A part of the plurality of optical transmitters may be a test optical transmitter, and may be configured to transmit test light (for example, an optical signal with a wavelength in a range greater than or equal to 492 nanometers and less than or equal to 577 nanometers). The other part of the plurality of optical transmitters may be a compensation optical transmitter, and may be configured to transmit compensation light (for example, an optical signal with a wavelength in a range greater than or equal to 1000 nanometers and less than or equal to 1500 nanometers).

The plurality of optical transmitters may directly transmit optical signals to the WDM 313, and then the WDM 313 may combine a plurality of optical signals with different wavelengths into one beam, and transmit the beam to the transmitter 316. Therefore, the plurality of optical signals with different wavelengths may be reflected by the reflector 316, to transmit along a same optical path to the skin surface of the user from the opening of the bottom wall 202 of the electronic device 100.

Optionally, the plurality of optical transmitters may be all connected to the waveguide. The plurality of optical signals with different wavelengths transmitted by the plurality of optical transmitters may be transmitted to the WDM 313 through the waveguide. The WDM 313 may be connected to the reflector 316 through the waveguide. The WDM 313 may combine the plurality of optical signals with different wavelengths into one beam, and transmit an optical signal after multiplexing along one waveguide to the reflector 316.

The reflector 316 may be a reflecting mirror, or may be a grating. A type of the reflecting mirror 316 is not limited in this embodiment of this application.

The electronic device may combine the test light and the compensation light into one beam by using the WDM, and transmit the beam to the skin surface of the user. Therefore, the electronic device can control the test light and the compensation light to have a same or similar optical path. Noise in a compensation PPG signal related to the compensation light may be the same as or similar to noise in a test PPG signal related to the test light. The electronic device can better reduce motion noise in the test PPG signal by using the compensation PPG signal, improving accuracy of heart rate detection.

In some embodiments, locations of the test optical transmitter and the compensation optical transmitter are close to each other. For example, a spacing between the location of the test optical transmitter and the location of the compensation optical transmitter is less than a preset distance threshold. Therefore, optical paths of the optical signals transmitted by the test optical transmitter and the compensation optical transmitter through the bottom wall of the electronic device may be similar to each other, so that a magnitude of the noise in the compensation PPG signal may be similar to a magnitude of the noise in the test PPG signal. This helps the electronic device better reduce the noise in the test PPG signal, improving accuracy of heart rate detection.

In some application scenarios, when the electronic device transmits an optical signal to perform heart rate detection, relative movement may be generated between the skin surface of the user and the bottom wall of the electronic device. For example, the electronic device is worn on a wrist of the user. In an application scenario of user motion, for example, running or playing basketball, a motion range of the wrist of the user is large. In consideration of wearing comfort, when the electronic device is worn, the bottom wall of the electronic device is usually not tightly attached to the skin of the user. Therefore, during user motion, the bottom wall of the electronic device may be deflected relative to the skin surface, that is, there is a deflection angle between the bottom wall and the skin surface.

Because deflection occurs between the electronic device and the skin surface of the user, the optical signal transmitted by the electronic device to the skin surface may also be deflected during transmission. Consequently, a reflected or scattered optical signal does not fall within a receiving range of the optical receiver, affecting the PPG signal generated by the optical receiver, and causing an inaccurate result of heart rate detection.

Embodiments of this application may provide a plurality of optical receivers, and by setting locations of the plurality of optical receivers on the electronic device, the plurality of optical receivers may receive reflected or scattered optical signals. The electronic device may select, based on strength of the optical signals received by the plurality of optical receivers, a PPG signal generated by at least one optical receiver, to calculate a heart rate. Therefore, the electronic device can reduce an error caused when the optical receiver does not receive a deflected optical signal, improving accuracy of heart rate detection.

The following describes an embodiment in which an electronic device reduces a heart rate detection error caused by deflection of the electronic device and a skin surface of a user.

FIG. 9 is an example schematic diagram of an application scenario in which deflection occurs between an electronic device and a skin surface of a user.

The electronic device may include at least one test optical transmitter 411, at least two test optical receivers (a first test optical receiver 412 and a second test optical receiver 413), a bottom wall, and a lens for covering an opening of the bottom wall. The electronic device may transmit an optical signal to the skin surface of the user through the lens, and receive a reflected or scattered optical signal through the lens.

A length of the test optical transmitter 411 may be d₁. For example, a value of d₁ may be specifically 0.05 millimeter. The test optical transmitter 411 may be configured to transmit test light (for example, an optical signal with a wavelength in a range greater than or equal to 492 nanometers and less than or equal to 577 nanometers).

A length of the first test optical receiver 412 and a length of the second test optical receiver 413 each may be d₂. For example, a value of d₂ may be specifically 0.1 millimeter.

The test optical transmitter 411, the length of the first test optical receiver 412, and the second test optical receiver 413 may be located on a same horizontal plane, and a distance from the bottom wall (that is, the lens) of the electronic device is d₃. For example, a value of d₃ may be specifically 0.2 millimeter. A width of the lens may be d₄. For example, a value of d₄ may be specifically 1 millimeter.

As shown in FIG. 9, there is a deflection angle α₁ between the bottom wall (that is, the lens) of the electronic device and the skin surface. The optical signal transmitted by the electronic device to the skin surface may be deflected during transmission.

Specifically, the test optical transmitter 411 may transmit test light with a beam width as a beam width shown in FIG. 9. An included angle between an optical signal 401a on an edge of the test light and a vertical direction may be α₂. For example, a value of α₂ may be specifically 10°.

When the test light is transmitted to the lens, the foregoing transmission direction of the test light may be deflected. An included angle between an optical signal 401b that is on an edge of the test light and that is transmitted to the lens and the vertical direction may be α₃. For example, a value of α₃ may be specifically 6.6°.

The foregoing test light may be transmitted to the skin surface of the user through the lens. If there is no deflection angle between the lens and the skin surface, that is, a plane on which the lens is located is close to and parallel to the skin surface, the test light may be transmitted to a human tissue in a direction indicated by a black dashed line shown in FIG. 9. Because there is the deflection angle α₁ between the lens and the skin surface, a direction in which the test light is transmitted to the human tissue is deflected, and the test light may be transmitted to the human tissue in a direction indicated by a black solid line shown in FIG. 9. An included angle between an optical signal 401c that is on an edge of the test light and that is transmitted to the human tissue and the vertical direction may be α₁ + α₃. The human tissue may include blood, a muscle, and a skeleton.

The test light may be absorbed, reflected, and scattered by the skin and the human tissue. If there is no deflection angle between the lens and the skin surface, the reflected or scattered optical signal may be transmitted to the first test optical receiver 412 in a direction indicated by a gray dashed line shown in FIG. 9. Therefore, the electronic device may calculate a heart rate based on a PPG signal generated by the first test optical receiver 412.

However, when there is the deflection angle between the lens and the skin surface, a transmission direction of the reflected or scattered optical signal is deflected, and the reflected or scattered optical signal may be transmitted in a direction indicated by a gray solid line shown in FIG. 9. The reflected or scattered optical signal being deflected does not fall within a receiving range of the first test optical receiver 412. The electronic device may be equipped with the second test optical receiver 413 to receive the reflected or scattered optical signal being deflected.

The second test optical receiver 413 and the first test optical receiver 412 may be located on a same side of the test optical transmitter 411. In addition, the second test optical receiver 413 may be located on a straight line on which the first test optical receiver 412 and the test optical transmitter 411 are located.

In this embodiment of this application, values of d₁, d₂, d₃, d₄, α₁, α₂, and α₃ are not limited.

In the application scenario shown in FIG. 9, the electronic device may include a plurality of optical receivers to receive the reflected or scattered optical signal. This reduces a case in which the optical receiver cannot receive the optical signal or can only receive a part of the optical signal due to deflection between the electronic device and the skin surface of the user. Therefore, the electronic device can reduce an error caused by the deflection between the electronic device and the skin surface of the user, improving accuracy of performing heart rate detection by using the PPG signal.

The electronic device may determine, based on strength of the optical signal received by the optical receiver, a PPG signal used for heart rate detection.

In some embodiments, the electronic device may select, based on strength of optical signals received by the plurality of optical receivers, such as the first test optical receiver 412 and the second test optical receiver 413, a PPG signal generated by at least one test optical receiver from the optical signals to perform heart rate detection.

For example, when strength of an optical signal received by the second test optical receiver 413 is far greater than strength of an optical signal received by the first test optical receiver 412, the electronic device may perform heart rate detection based on an optical signal generated by the second test optical receiver 413. The strength of the optical signal received by the second test optical receiver 413 is far greater than the strength of the optical signal received by the first test optical receiver 412. This may indicate that a deflection angle is generated between the bottom wall of the electronic device and the skin surface of the user, and a transmission direction of the optical signal is deflected.

Optionally, both the first test optical receiver 412 and the second test optical receiver 413 receive some reflected or refracted optical signals. The electronic device may add up PPG signals generated by the first test optical receiver 412 and the second test optical receiver 413, and then calculate the heart rate based on a PPG signal obtained through addition.

The electronic device may determine, by using an accelerometer, a PPG signal used for heart rate detection.

In some embodiments, the accelerometer may be disposed in the electronic device. The electronic device may determine, by using the accelerometer, deflection between the electronic device and the skin surface of the user, to further determine the PPG signal used for heart rate detection.

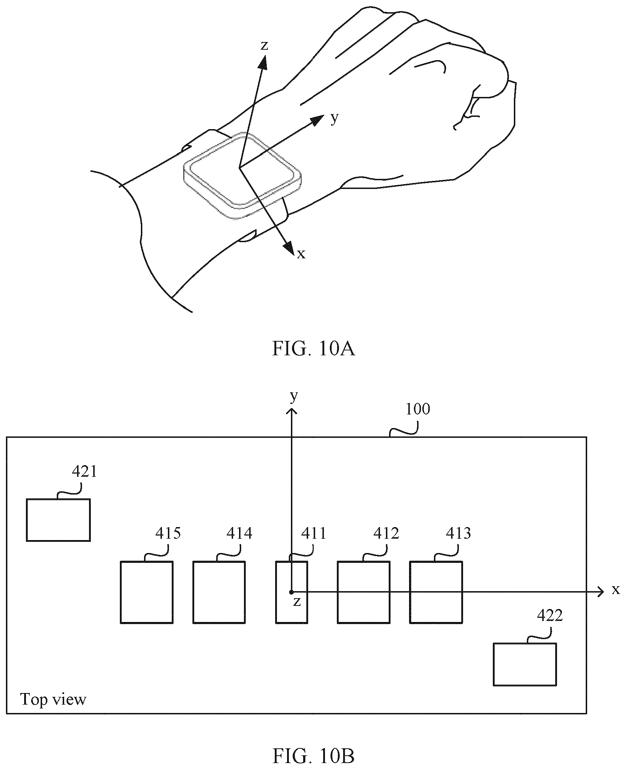

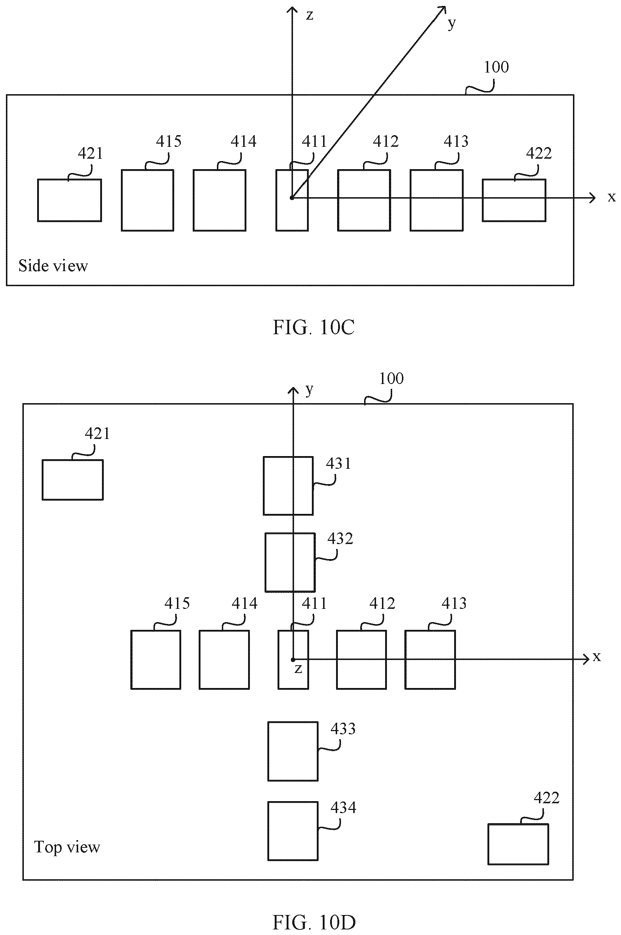

Each of FIG. 10A, FIG. 10B, and FIG. 10C is an example schematic diagram of a structure of an electronic device that uses an accelerometer to determine a PPG signal used for heart rate detection. FIG. 10B and FIG. 10C are respectively a top view and a side view of the electronic device 100.

As shown in FIG. 10A, the electronic device 100 may be a smartwatch. When the electronic device 100 is worn on the wrist of the user, the electronic device 100 may establish a right-handed rectangular coordinate system shown in FIG. 10A. A y-axis may be an axis along a plane on which the bottom wall of the electronic device 100 is located and parallel to a direction of the arm of the user. An x-axis may be an axis along the plane on which the bottom wall of the electronic device 100 is located and perpendicular to the direction of the arm of the user. A z-axis may be an axis perpendicular to the plane on which the bottom wall of the electronic device 100 is located. A positive direction of the z-axis may be a direction from the bottom wall of the electronic device 100 toward an upper surface of the electronic device 100 (that is, a side on which a display screen is located). A plane determined by the x-axis and the y-axis may be the plane on which the bottom wall of the electronic device 100 is located.

As shown in FIG. 10B and FIG. 10C, the electronic device 100 may include a test optical transmitter 411, a first test optical receiver 412, a second test optical receiver 413, a third test optical receiver 414, a fourth test optical receiver 415, a first accelerometer 421, and a second accelerometer 422. The test optical transmitter 411, the first test optical receiver 412, the second test optical receiver 413, the third test optical receiver 414, and the fourth test optical receiver 415 may be located on one straight line. The first accelerometer 421 and the second accelerometer 422 may be respectively disposed on two sides of the straight line on which the test optical transmitter 411 and each test optical receiver are located. As shown in FIG. 10B, the first accelerometer 421 may be disposed on one side of the straight line, and a spacing between the first accelerometer 421 and the fourth test optical receiver 415 is less than a first spacing. The second accelerometer 422 may be disposed on the other side of the straight line, and a spacing between the second accelerometer 422 and the second test optical receiver 413 is less than a second spacing. Both the first spacing and the second spacing are positive numbers, and neither the first spacing nor the second spacing is limited in this embodiment of this application.

In some other embodiments, the first accelerometer 421 and the second accelerometer 422 may be disposed on a same side of the straight line on which the test optical transmitter 411 and each test optical receiver are located.

The first accelerometer 421 and the second accelerometer 422 may be configured to detect whether the electronic device 100 is in a motion state. When the electronic device 100 is in the motion state, acceleration data in the first accelerometer 421 and the second accelerometer 422 is not zero or an absolute value of the acceleration data is far greater than zero.

When the electronic device 100 is not in the motion state, the electronic device 100 may calculate a heart rate based on a PPG signal generated by the first test optical receiver 412 and/or the third test optical receiver 414. When the electronic device 100 is in the motion state, the first accelerometer 421 may determine acceleration data (Ax1, Ay1, and Az1) of the electronic device 100 on three direction axes in the coordinate system shown in FIG. 10A. The second accelerometer 422 may determine acceleration data (Ax2, Ay2, and Az2) of the electronic device 100 on the three direction axes in the coordinate system shown in FIG. 10A.

If the acceleration data of the first accelerometer 421 on the z-axis is greater than the acceleration data of the second accelerometer 422 on the z-axis, the electronic device 100 is in the motion state, and a speed at which a side on which the first accelerometer 421 is located moves along the z-axis is higher than a speed at which a side on which the second accelerometer 422 is located moves along the z-axis. Further, a distance between the side on which the first accelerometer 421 is located and the skin surface of the user is greater than a distance between the side on which the second accelerometer 422 is located and the skin surface of the user, that is, there is a deflection angle between the bottom wall of the electronic device 100 and the skin surface of the user.

Refer to the deflection direction of the optical signal when there is the deflection angle between the electronic device and the skin surface of the user shown in FIG. 9. It can be learned that when the first accelerometer 421 is farther away from the skin surface of the user, an optical signal reflected or scattered by the human tissue and the skin may be deflected toward a direction in which the fourth test optical receiver 415 is located. The fourth test optical receiver 415 may receive the reflected or scattered optical signal after being deflected.

When a value of Az1 - Az2 is greater than a first preset threshold, the electronic device 100 may calculate the heart rate based on a PPG signal generated by the fourth test optical receiver 415. Alternatively, the electronic device may add up the PPG signal generated by the fourth test optical receiver 415 and the PPG signal generated by the third test optical receiver 414, and calculate the heart rate based on a PPG signal obtained through addition.

The first preset threshold may be a value approaching zero and greater than zero, and a specific value of the first preset threshold is not limited in this embodiment of this application.

Similarly, a value of Az2 - Az1 is greater than the first preset threshold. This may indicate that the side on which the second accelerometer 422 is located is farther away from the skin surface of the user, and the optical signal reflected or scattered by the human tissue and the skin may be deflected toward a direction in which the second test optical receiver 413 is located. The second test optical receiver 413 may receive the transmitted or scattered optical signal after being deflected.

When the value of Az2 - Az1 is greater than the first preset threshold, the electronic device 100 may calculate the heart rate based on a PPG signal generated by the second test optical receiver 413. Alternatively, the electronic device may add up the PPG signal generated by the second test optical receiver 413 and the PPG signal generated by the first test optical receiver 412, and calculate the heart rate based on a PPG signal obtained through addition.

Test optical receivers in the electronic device 100 may not be limited to the four test optical receivers: the first test optical receiver 412, the second test optical receiver 413, the third test optical receiver 414, and the fourth test optical receiver 415. In the electronic device 100 shown in FIG. 10B, the right side of the test optical transmitter 411 (that is, a side in a positive direction along the x-axis) may include three, four, ..., and M test optical receivers. The left side of the test optical transmitter 411 (that is, a side in an opposite direction along the x-axis) may also include three, four, ..., and M test optical receivers, where M is an integer greater than 2. The plurality of test optical receivers on the right side and left side of the test optical transmitter 411 and the test optical transmitter 411 may be located on one straight line.

In a possible implementation, the electronic device is a smartwatch or a smart band. The electronic device includes a watchband. A plurality of pinholes are provided on the watchband. The plurality of pinholes may be used to adjust a circumference of the watchband when the smartwatch or the smart band is worn. A distance between two adjacent test optical receivers in the electronic device, for example, a distance between the first test optical receiver 412 and the second test optical receiver 413, or a distance between the third test optical receiver 414 and the fourth test optical receiver 415, may be determined by a spacing between two adjacent pinholes.

Specifically, the spacing between two adjacent pinholes on the watchband of the electronic device is a. The distance between two adjacent test optical receivers in the electronic device may be µ * λ * a, where λ may represent that the electronic device uses a spacing between two adjacent pinholes in λ pinholes to determine the distance between two adjacent test optical receivers, λ is a positive integer, for example, 1 or 2, and µ may be a positive number greater than 0 and less than or equal to 1.

In this embodiment of this application, a distance between the test optical transmitter 411 and the first test optical receiver 412 is not limited, and a distance between the test optical transmitter 411 and the third test optical receiver 414 is not limited.

A distance between the test optical receivers is not limited in this embodiment of this application.

In some embodiments, the plurality of test optical receivers in the electronic device 100 may be disposed at locations shown in FIG. 10D.

As shown in FIG. 10D, the electronic device 100 may include a test optical transmitter 411, a first test optical receiver 412, a second test optical receiver 413, a third test optical receiver 414, a fourth test optical receiver 415, a fifth test optical receiver 431, a sixth test optical receiver 432, a seventh test optical receiver 433, an eighth test optical receiver 434, a first accelerometer 421, and a second accelerometer 422.

The test optical transmitter 411, the first test optical receiver 412, the second test optical receiver 413, the third test optical receiver 414, and the fourth test optical receiver 415 may be located on a first straight line. The test optical transmitter 411, the fifth test optical receiver 431, the sixth test optical receiver 432, the seventh test optical receiver 433, and the eighth test optical receiver 434 may be located on a second straight line. The first straight line may be perpendicular to the second straight line.

On the first straight line, the electronic device 100 may be further equipped with more or fewer test optical receivers on both sides of the test optical transmitter 411. On the second straight line, the electronic device 100 may be further equipped with more or fewer test optical receivers on both sides of the test optical transmitter 411.

The first right angle and the second straight line may divide the electronic device into four regions: a first region, a second region, a third region, and a fourth region. The first region may be a region close to the second test optical receiver 413 and the fifth test optical receiver 431. The second region may be a region close to the fourth test optical receiver 415 and the fifth test optical receiver 431. The third region may be a region close to the fourth test optical receiver 415 and the eighth test optical receiver 434. The fourth region may be a region close to the second test optical receiver 413 and the eighth test optical receiver 434.

The first accelerometer 421 and the second accelerometer 422 may be respectively disposed in the second region and the fourth region. Alternatively, the first accelerometer 421 and the second accelerometer 422 may be respectively disposed in the first region and the third region.

As shown in FIG. 10D, when the acceleration data Az1 of the first accelerometer 421 on the z-axis is greater than the acceleration data Az2 of the second accelerometer 422 on the z-axis, and a distance between a side on which the first accelerometer 421 is located and the skin surface of the user is greater than a distance between the second accelerometer 422 and the skin surface of the user, the optical signal reflected or scattered by the human tissue and the skin may be deflected toward a direction in which the fourth test optical receiver 415 and the fifth test optical receiver 431 are located. The fourth test optical receiver 415 and the fifth test optical receiver 431 may receive the reflected or scattered optical signal being deflected.

When a value of Az1 - Az2 is greater than a first threshold, the electronic device 100 may calculate the heart rate based on a PPG signal generated by the fourth test optical receiver 415 and a PPG signal generated by the fifth test optical receiver 431. The electronic device 100 may select, based on strength of optical signals received by the fourth test optical receiver 415 and the fifth test optical receiver 431, the PPG signal generated by one of the optical receivers to calculate the heart rate.

Optionally, the electronic device 100 may add up the PPG signal generated by the fourth test optical receiver 415 and the PPG signal generated by the fifth test optical receiver 431, and calculate the heart rate based on a PPG signal obtained through addition.

Optionally, the electronic device 100 may add up a PPG signal generated by the third test optical receiver 414, the PPG signal generated by the fourth test optical receiver 415, the PPG signal generated by the fifth test optical receiver 431, and a PPG signal generated by the sixth test optical receiver 432, and calculate the heart rate based on a PPG signal obtained through addition.

Similarly, when the acceleration data Az2 of the second accelerometer 422 on the z-axis is greater than the acceleration data Az1 of the first accelerometer 421 on the z-axis, and a distance between a side on which the second accelerometer 422 is located and the skin surface of the user is greater than a distance between the first accelerometer 421 and the skin surface of the user, the optical signal reflected or scattered by the human tissue and the skin may be deflected toward a direction in which the second test optical receiver 413 and the eighth test optical receiver 434 are located. The second test optical receiver 413 and the eighth test optical receiver 434 may receive the reflected or scattered optical signal being deflected.

When a value of Az2 - Az1 is greater than the first threshold, the electronic device 100 may calculate the heart rate based on the following one or more PPG signals and according to the method mentioned in the foregoing embodiments: a PPG signal generated by the first test optical receiver 412, a PPG signal generated by the second test optical receiver 413, a PPG signal generated by the seventh test optical receiver 433, and a PPG signal generated by the eighth test optical receiver 434.

The electronic devices shown in FIG. 10B, FIG. 10C, and FIG. 10D are not limited to including one test optical transmitter. The electronic device may include a plurality of test optical transmitters. Locations of the one or more test optical transmitters are not limited in this embodiment of this application.

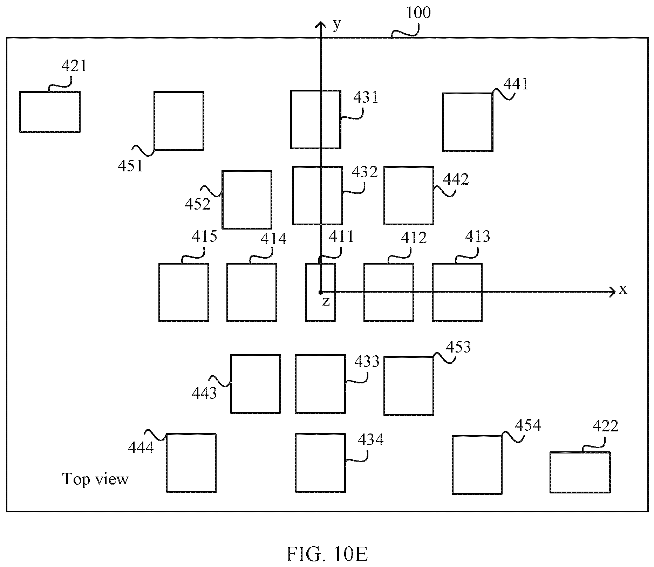

In addition to disposing the test optical receivers in arrangement manners shown in FIG. 10B and FIG. 10D, the electronic device may be further equipped with the plurality of test optical receivers in another arrangement manner. For example, the plurality of test optical receivers may be arranged based on straight line distribution shown in an "asterisk-shaped" structure in FIG. 10E, and a plurality of test optical receivers may be included on each straight line.

As shown in FIG. 10E, the test optical receivers 412 to 415 may be included on a first straight line in which the x-axis is located. The test optical receivers 431 to 434 may be included on a second straight line in which the y-axis is located. Test optical receivers 441 to 444 may be included on a third straight line that is at an angle of 45° with both the x-axis and the y-axis and that passes through the first quadrant and the third quadrant of a coordinate system on which the x-axis and the y-axis are located. Test optical receivers 451 to 454 may be included on a fourth straight line that is at an angle of 45° with both the x-axis and the y-axis, and that passes through the second quadrant and the fourth quadrant of the coordinate system on which the x-axis and the y-axis are located. The test optical transmitter 411 may be located at a location at which the first straight line, the second straight line, the third straight line, and the fourth straight line intersect.

The electronic device may determine a deflection direction of the electronic device according to the method in the embodiment in FIG. 10D, and then determine to use PPG signals generated by which of the test optical receivers 412 to 415 on the first straight line and the test optical receivers 431 to 434 on the second straight line to calculate the heart rate.

In addition, the electronic device may determine, with reference to the acceleration data of the first accelerometer 421 and the second accelerometer 422 on the x-axis and the y-axis, to use PPG signals generated by which of the test optical receivers 441 to 444 on the third straight line and the test optical receivers 451 to 454 on the fourth straight line to calculate the heart rate.

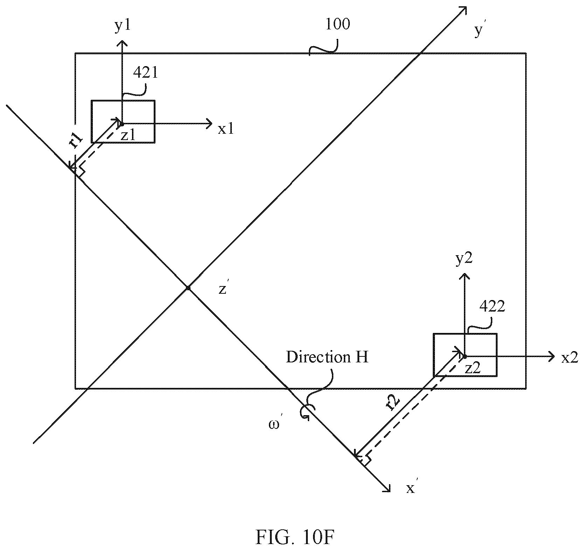

Specifically, as shown in FIG. 10F, a coordinate system that uses a location of the first accelerometer as an origin is x1-y1-z1, and a coordinate system that uses a location of the second accelerometer as an origin is x2-y2-z2. Directions of the x1-axis and the x2-axis may be the same as a direction of the x-axis shown in FIG. 10A. Directions of the y1-axis and the y2-axis may be the same as a direction of the y-axis shown in FIG. 10A. Directions of the z1-axis and the z2-axis may be the same as a direction of the z-axis shown in FIG. 10A. In a coordinate system x'-y'-z', directions of an x'-axis and a y'-axis may be respectively directions of the x1-axis and the y 1-axis after the coordinate system x1-y1-z1 is rotated 45° clockwise around the z1-axis, (that is, respectively directions of the x2-axis and the y2-axis after the coordinate system x2-y2-z2 is rotated 45° clockwise around the z2-axis), and a z'-axis may be in a same direction as the z1-axis (that is, in a same direction as the z2-axis).

The electronic device may deflect around the x'-axis in a direction H shown in FIG. 10F, a deflection angle is θ, and a deflection angular velocity is ω'.

Then, a transient acceleration of the first accelerometer 421 is a1 = ω' * r1. A transient acceleration of the second accelerometer 422 is a2 = ω' * r2. An acceleration component of the first accelerometer 421 on the z'-axis is Az1 = a1 * cos (θ). An acceleration component of the second accelerometer 422 on the z'-axis is Az2 = a2 * cos (θ). An acceleration component of the first accelerometer 421 on a plane on which the x'-axis and the y'-axis are located is Axy1 = a1 * sin(θ). An acceleration component of the second accelerometer 422 on the plane on which the x'-axis and the y'-axis are located is Axy2 = a2 * sin(θ).

Further, the electronic device may learn that accelerations of the first accelerometer 421 on the x'-axis and the y'-axis are respectively Ax1 = a1 * sin(θ) * cos(45°) and Ay1 = a1 * sin(θ) * cos (45°), and accelerations of the second accelerometer 422 on the x'-axis and the y'-axis are respectively Ax2 = a1 * sin(θ) * cos(45°) and Ay2 = a1 * sin(θ) * cos (45°).

It can be learned from the foregoing analysis that, Ax1 = Ay1, Ax2 = Ay2, and Ax1/Ax2 = Ay1/Ay2 = r1/r2. The electronic device deflects around the x'-axis in the direction H shown in FIG. 10F, and Ax1, Ax2, Ay1, and Ay2 may be negative values. The electronic device deflects around the x'-axis in an opposite direction to the direction H shown in FIG. 10F, and Ax1, Ax2, Ay1, and Ay2 may be positive values.

For the test optical transmitter and the test optical receiver that are arranged in the "asterisk-shaped" structure shown in FIG. 10E, when acceleration data Ax1 of the first accelerometer 421 on the x-axis is the same as or similar to acceleration data Ay1 thereof on the y-axis, acceleration data Ax2 of the second accelerometer 422 on the x-axis is the same as or similar to acceleration data Ay2 thereof on the y-axis, and a value of Ax1/Ax2 is the same as or similar to a value of Ay1/Ay2, the electronic device may determine that the deflection direction of the electronic device is a direction of the third straight line.

When Ax1, Ax2, Ay1, and Ay2 are negative values, the electronic device may determine that a distance between the test optical receiver 441 and the skin surface of the user is farther than a distance between the test optical receiver 442 and the skin surface of the user. The electronic device may calculate the heart rate by using a PPG signal generated by the test optical receiver 441. Alternatively, the electronic device may calculate the heart rate by using a sum of the PPG signal generated by the test optical receiver 441 and a PPG signal generated by the test optical receiver 442.

When Ax1, Ax2, Ay1, and Ay2 are positive values, the electronic device may determine that a distance between the test optical receiver 444 and the skin surface of the user is farther than a distance between the test optical receiver 443 and the skin surface of the user. The electronic device may calculate the heart rate by using a PPG signal generated by the test optical receiver 444. Alternatively, the electronic device may calculate the heart rate by using a sum of a PPG signal generated by the test optical receiver 443 and the PPG signal generated by the test optical receiver 444.

Refer to the embodiment shown in FIG. 10F. The electronic device may determine to use PPG signals generated by which of the test optical receivers 451 to 454 on the fourth straight line to calculate the heart rate. Details are not described herein again.

In some embodiments, in addition to 45°, there may be another included angle between the third straight line and each of the x-axis and the y-axis, and there may be another included angle between the fourth straight line and each of the x-axis and the y-axis. Refer to the embodiment shown in FIG. 10F. The electronic device may determine to use PPG signals generated by which test optical receivers on the third straight line and the fourth straight line to calculate the heart rate. Details are not described herein again.

In some embodiments, the electronic device may include at least one test optical receiver and a plurality of test optical transmitters. Locations of the plurality of test optical transmitters in the electronic device may be locations of the plurality of test optical receivers shown in FIG. 10B. A location of the at least one test optical receiver in the electronic device may be a location of the test optical transmitter 411 shown in FIG. 10B. In other words, the plurality of test optical transmitters and the at least one test optical receiver may be located on one straight line. Refer to the embodiment shown in FIG. 10B. The electronic device may determine, by using the accelerometer, to drive which test optical transmitters to transmit optical signals, and calculate the heart rate by using a PPG signal generated by the at least one test optical receiver.

Alternatively, locations of the plurality of test optical transmitters in the electronic device may be locations of the plurality of test optical receivers shown in FIG. 10D. A location of the at least one test optical receiver in the electronic device may be a location of the test optical transmitter 411 shown in FIG. 10D. In other words, the plurality of test optical transmitters may be distributed in two perpendicularly intersecting straight lines, and the at least one test optical receiver may be located at an intersection point of the two perpendicularly intersecting straight lines. Refer to the embodiment shown in FIG. 10D. The electronic device may determine, by using the accelerometer, to drive which test optical transmitters to transmit optical signals, and calculate the heart rate by using a PPG signal generated by the at least one test optical receiver.

Alternatively, locations of the plurality of test optical transmitters in the electronic device may be locations of the plurality of test optical receivers shown in FIG. 10E. A location of the at least one test optical receiver in the electronic device may be a location of the test optical transmitter 411 shown in FIG. 10E. In other words, the plurality of test optical transmitters and the at least one test optical receiver may be arranged in an "asterisk-shaped" structure. Refer to the embodiment shown in FIG. 10E. The electronic device may determine, by using the accelerometer, to drive which test optical transmitters to transmit optical signals, and calculate the heart rate by using a PPG signal generated by the at least one test optical receiver.

The plurality of test optical transmitters and the at least one test optical receiver may be alternatively located in other locations in the electronic device. This is not limited in this embodiment of this application.

The electronic device may be equipped with a plurality of optical receivers to receive reflected or scattered optical signals, and select, from PPG signals generated by the plurality of optical receivers and based on strength of the optical signals received by the optical receivers and/or acceleration data detected by the accelerometer, a PPG signal generated by at least one optical receiver, to perform heart rate detection. Therefore, the electronic device can reduce an error caused by the deflection between the electronic device and the skin surface of the user, improving accuracy of performing heart rate detection by using the PPG signal.

In an embodiment of this application, a first optical transmitter may be the test optical transmitter mentioned in the foregoing embodiments. A first optical signal transmitted by the first optical transmitter may be the test light mentioned in the foregoing embodiments. A second optical transmitter may be the compensation optical transmitter mentioned in the foregoing embodiments. A second optical signal transmitted by the second optical transmitter may be compensation light.

A first optical receiver may be the test optical receiver mentioned in the foregoing embodiments. A third optical signal received by the first optical receiver may be test light formed through reflection or scattering of the first optical signal by a human tissue (for example, blood, a skin, a muscle, or a skeleton). A second optical receiver may be the compensation optical receiver mentioned in the foregoing embodiments. A fourth optical signal received by the second optical receiver may be compensation light formed through reflection or scattering of the second optical signal by the human tissue.

A first PPG signal may be the first test PPG signal whose electrical parameter is a current mentioned in the foregoing embodiments. A second PPG signal may be the first compensation PPG signal whose electrical parameter is a voltage mentioned in the foregoing embodiments. The electronic device obtains a third PPG signal based on the first PPG signal and the second PPG signal. The electronic device may first convert the first PPG signal into a fourth PPG signal (that is, the second test PPG signal whose electrical parameter is a voltage mentioned in the foregoing embodiments), and convert the second PPG signal into a fifth PPG signal (that is, the second compensation PPG signal whose electrical parameter is a voltage mentioned in the foregoing embodiments). Further, the electronic device may perform an operation of subtracting the second compensation PPG signal from the second test PPG signal to obtain the third PPG signal. Alternatively, the first optical receiver and the second optical receiver in the electronic device are connected in series. When the first optical receiver generates the first PPG signal, and the second optical receiver generates the second PPG signal, some current signals in the first PPG signal may flow to the second optical receiver. Strength of the some current signals flowing to the second optical receiver is the same as strength of the second PPG signal. A current signal in the first PPG signal that does not flow to the second optical receiver is a sixth PPG signal. The electronic device may convert the sixth PPG signal into a voltage signal, and the voltage signal may be the third PPG signal.

The third PPG signal may be equivalent to a noise-reduced signal. The electronic device may obtain a heart rate by using the third PPG signal, improving accuracy of heart rate detection.

In this embodiment of this application, a fifth optical signal may be an optical signal obtained after the wavelength division multiplexer performs multiplexing processing on the first optical signal and the second optical signal. In other words, the fifth optical signal includes the first optical signal and the second optical signal. Therefore, optical paths of the first optical signal and the second optical signal may be consistent with each other in a transmission process. The fifth optical signal may be received by the first optical receiver and the second optical receiver after being reflected or scattered by the human tissue. The first optical receiver may generate the first PPG signal based on the first optical signal included in the received fifth optical signal. The second optical receiver may generate the second PPG signal based on the second optical signal included in the received fifth optical signal. Because the optical paths for transmitting the first optical signal and the second optical signal are consistent with each other, and a user may have same or similar impact on the first optical signal and the second optical signal during motion, a magnitude of motion noise included in the first PPG signal may be the same as or similar to a magnitude of motion noise included in the second PPG signal. Therefore, the electronic device can better reduce the magnitude of the motion noise in the first PPG signal.

In this embodiment of this application, a first receiver, a second receiver, a third receiver, and a fourth receiver may be the test optical receivers mentioned in the foregoing embodiments, and may respectively generate a first signal, a second signal, a third signal, and a fourth signal based on the first optical signal (that is, the test light). The first signal, the second signal, the third signal, and the fourth signal each may be the test PPG signal mentioned in the foregoing embodiments. For example, the first receiver may be the first test optical receiver 412 shown in FIG. 10B. The second receiver may be the second test optical receiver 413 shown in FIG. 10B. The third receiver may be the third test optical receiver 414 shown in FIG. 10B. The fourth receiver may be the fourth test optical receiver 415 shown in FIG. 10B. A first accelerometer may be the first accelerometer 421 shown in FIG. 10B. A second accelerometer may be the second accelerometer 422 shown in FIG. 10B. A first direction may be the positive direction of the z-axis shown in FIG. 10A. A first acceleration difference may be the first preset threshold mentioned in the foregoing embodiments. The first preset threshold may be a value approaching zero and greater than zero, and a specific value of the first preset threshold is not limited in this embodiment of this application.

According to the context, the term "when..." used in the foregoing embodiments may be interpreted as a meaning of "if...", "after...", "in response to determining...", or "in response to detecting...". Similarly, according to the context, the phrase "when it is determined that..." or "if (a stated condition or event) is detected" may be interpreted as a meaning of "if it is determined that... ", "in response to determining... ", "when (a stated condition or event) is detected", or "in response to detecting (a stated condition or event)".

All or some of the foregoing embodiments may be implemented by using software, hardware, firmware, or any combination thereof. When software is used to implement the foregoing embodiments, all or some of the foregoing embodiments may be implemented in a form of a computer program product. The computer program product includes one or more computer instructions. When the computer program instructions are loaded and executed on a computer, all or some of the procedures or the functions according to embodiments of this application are generated. The computer may be a general-purpose computer, a dedicated computer, a computer network, or another programmable apparatus. The computer instructions may be stored in the computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by a computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive), or the like.

A person of ordinary skill in the art may understand that all or some of the procedures of the methods in the foregoing embodiments may be implemented by a computer program instructing related hardware. The program may be stored in a computer-readable storage medium. When the program is executed, the procedures of the methods in the foregoing embodiments may be performed. The storage medium includes any medium that can store program code, for example, a ROM, a random access memory RAM, a magnetic disk, or an optical disc.

## Claims

1. A method for obtaining a heart rate, applied to an electronic device (100), wherein the electronic device (100) comprises a bottom wall (202), at least one first optical transmitter (211A), at least one second optical transmitter (211B), at least one first optical receiver (212A), and at least one second optical receiver (212B), a first opening and a second opening are provided on the bottom wall (202), optical signals transmitted by the first optical transmitter (211A) and the second optical transmitter (211B) are transmitted from the bottom wall (202) through the first opening, the first optical receiver (212A) and the second optical receiver (212B) receive optical signals through the second opening, the first optical receiver (212A) includes a first optical filter, and the second optical receiver (212B) includes a second optical filter;
the method comprises:
driving, by the electronic device (100), the first optical transmitter (211A) to transmit a first optical signal (211a) with a first wavelength, and driving the second optical transmitter (211B) to transmit a second optical signal (211b) with a second wavelength, wherein the first wavelength is greater than or equal to 492 nanometers and less than or equal to 577 nanometers, and the second wavelength is greater than or equal to 1000 nanometers and less than or equal to 1500 nanometers;
receiving, by the electronic device (100), a third optical signal by using the first optical receiver (212A), and obtaining a first PPG signal based on the third optical signal, wherein the third optical signal is formed through reflection or scattering of the first optical signal (211a), and the first optical filter in the first optical receiver (212A) filters reflection or scattering of the second optical signal (211b);
receiving, by the electronic device (100), a fourth optical signal by using the second optical receiver (212B), and obtaining a second PPG signal based on the fourth optical signal, wherein the fourth optical signal is formed through reflection or scattering of the second optical signal (211b), and the second optical filter in t he second optical receiver (212B) filters reflection or scattering of the first optical signal (211a);
obtaining, by the electronic device (100), a third PPG signal by performing subtraction processing on the first PPG signal and the second PPG signal; and
obtaining, by the electronic device (100), a heart rate by using the third PPG signal.

2. The method according to claim 1, wherein the electronic device (100) further comprises a wavelength division multiplexer (313) and a reflector (314), the wavelength division multiplexer (313) comprises a first light incident surface, a second light incident surface, and a first light emergent surface, the first light incident surface is opposite to a light emergent surface of the first optical transmitter (211A), the second light incident surface is opposite to a light emergent surface of the second optical transmitter (211B), and the method comprises:
driving, by the electronic device (100), the first optical transmitter (211A) to transmit the first optical signal to the first light incident surface, and driving the second optical transmitter (211B) to transmit the second optical signal to the second light incident surface, wherein the first optical signal and the second optical signal are received by the wavelength division multiplexer (313); and
performing, by the electronic device (100), multiplexing processing on the first optical signal and the second optical signal by using the wavelength division multiplexer (313), to obtain a fifth optical signal, wherein the fifth optical signal is transmitted from the first light emergent surface to the reflector, and is transmitted from the bottom wall after being reflected by the reflector.

3. The method according to claim 2, wherein the electronic device (100) further comprises a first waveguide, a second waveguide, and a third waveguide, the first waveguide is configured to connect the light emergent surface of the first optical transmitter (211A) to the first light incident surface, the second waveguide is configured to connect the light emergent surface of the second optical transmitter (211B) to the second light incident surface, and the third waveguide is configured to connect the first light emergent surface to the reflector, and the method further comprises:
transmitting, by the electronic device (100), the first optical signal from the first optical transmitter (211A) to the wavelength division multiplexer (313) by using the first waveguide;
transmitting, by the electronic device (100), the second optical signal from the second optical transmitter (211B) to the wavelength division multiplexer (313) by using the second waveguide; and
transmitting, by the electronic device (100), the fifth optical signal from the wavelength division multiplexer (313) to the reflector by using the third waveguide.

4. The method according to any one of claims 1 to 3, wherein the obtaining, by the electronic device (100), a third PPG signal based on the first PPG signal and the second PPG signal specifically comprises:
converting, by the electronic device (100), the first PPG signal into a fourth PPG signal, and converting the second PPG signal into a fifth PPG signal, wherein both the first PPG signal and the second PPG signal are current signals, and both the fourth PPG signal and the fifth PPG signal are voltage signals; and
subtracting, by the electronic device (100), the fifth PPG signal from the fourth PPG signal, to obtain the third PPG signal.

5. The method according to any one of claims 1 to 3, wherein the first optical receiver (212A) is connected in series to the second optical receiver (212B), both the first PPG signal and the second PPG signal are current signals, some current signals in the first PPG signal flow to the second optical receiver (212B), strength of the some current signals is the same as strength of the second PPG signal, and the method comprises:
obtaining, by the electronic device (100), a sixth PPG signal by using the first optical receiver (212A), wherein strength of the sixth PPG signal is the same as strength of a current signal that is in the first PPG signal and that does not flow to the second optical receiver (212B); and
converting, by the electronic device (100), the sixth PPG signal into a voltage signal, to obtain the third PPG signal, wherein the sixth PPG signal is a current signal.

6. The method according to claim 1, wherein the first optical receiver (212A) and the second optical receiver (212B) are a same optical receiver, the driving, by the electronic device (100), the first optical transmitter (211A) to transmit a first optical signal with a first wavelength, and driving the second optical transmitter (211B) to transmit a second optical signal with a second wavelength, the receiving, by the electronic device (100), a third optical signal by using the first optical receiver (212A), and obtaining a first PPG signal based on the third optical signal, and the receiving, by the electronic device (100), a fourth optical signal by using the second optical receiver (212B), and obtaining a second PPG signal based on the fourth optical signal specifically comprise:
driving, by the electronic device (100) in a first time period, the first optical transmitter (211A) to transmit the first optical signal;
obtaining, by the electronic device (100), the first PPG signal based on the third optical signal received by the optical receiver;
driving, by the electronic device (100) in a second time period, the second optical transmitter (211B) to transmit the second optical signal; and
obtaining, by the electronic device (100), the second PPG signal based on the fourth optical signal received by the optical receiver, wherein
an interval between the first time period and the second time period is less than first duration.

7. An electronic device (100), comprising a bottom wall, at least one first optical transmitter (211A), at least one second optical transmitter (211B), at least one first optical receiver (212A), at least one second optical receiver (212B), an analog front end (220), and a processor (230), wherein a first opening and a second opening are provided on the bottom wall, optical signals transmitted by the first optical transmitter (211A) and the second optical transmitter (211B) are transmitted from the bottom wall through the first opening, the first optical receiver (212A) and the second optical receiver (212B) receive optical signals through the second opening, the first optical receiver (212A) includes a first optical filter, and the second optical receiver (212B) includes a second optical filter;
the first optical transmitter (211A) is configured to transmit a first optical signal with a first wavelength, wherein the first wavelength is greater than or equal to 492 nanometers and less than or equal to 577 nanometers;
the second optical transmitter (211B) is configured to transmit a second optical signal with a second wavelength, wherein the second wavelength is greater than or equal to 1000 nanometers and less than or equal to 1500 nanometers;
the first optical receiver (212A) is configured to receive a third optical signal, and generate a first PPG signal based on the third optical signal, wherein the third optical signal is formed through reflection or scattering of the first optical signal, and the first optical filter in the first optical receiver (212A) **is** configured to filter reflection or scattering of the second optical signal (211b);
the second optical receiver (212B) is configured to receive a fourth optical signal, and generate a second PPG signal based on the fourth optical signal, wherein the fourth optical signal is formed through reflection or scattering of the second optical signal, and the second optical filter in the second optical receiver (212B) is configured to filter reflection or scattering of the first optical signal (211a);
the analog front end (220) is configured to obtain a third PPG signal by performing subtraction processing on the first PPG signal and the second PPG signal; and
the processor (230) is configured to obtain a heart rate by using the third PPG signal.

8. The electronic device (100) according to claim 7, wherein the electronic device (100) further comprises a wavelength division multiplexer (313) and a reflector (314), and the wavelength division multiplexer (313) comprises a first light incident surface, a second light incident surface, and a first light emergent surface;
the first light incident surface is opposite to a light emergent surface of the first optical transmitter (211A), and is configured to receive an optical signal transmitted by the first optical transmitter (211A);
the second light incident surface is opposite to a light emergent surface of the second optical transmitter (211B), and is configured to receive an optical signal transmitted by the second optical transmitter (211B); and
the reflector is configured to receive an optical signal transmitted from the first light emergent surface, and transmit, through the bottom wall, the optical signal transmitted from the first light emergent surface.

9. The electronic device (100) according to claim 8, wherein the electronic device (100) further comprises a first waveguide, a second waveguide, and a third waveguide;
the first waveguide is configured to connect the light emergent surface of the first optical transmitter (211A) to the first light incident surface, to transmit the first optical signal to the wavelength division multiplexer (313);
the second waveguide is configured to connect the light emergent surface of the second optical transmitter (211B) to the second light incident surface, to transmit the second optical signal to the wavelength division multiplexer (313); and
the third waveguide is configured to connect the first light emergent surface to the reflector, to transmit, to the reflector, the optical signal transmitted from the first light emergent surface.

10. The electronic device (100) according to any one of claims 7 to 9, wherein the analog front end (220) is specifically configured to:
convert the first PPG signal into a fourth PPG signal, and convert the second PPG signal into a fifth PPG signal, wherein both the first PPG signal and the second PPG signal are current signals, and both the fourth PPG signal and the fifth PPG signal are voltage signals; and
subtract the fifth PPG signal from the fourth PPG signal, to obtain the third PPG signal.

11. The electronic device (100) according to any one of claims 7 to 9, wherein the first optical receiver (212A) is connected in series to the second optical receiver (212B), both the first PPG signal and the second PPG signal are current signals, some current signals in the first PPG signal flow to the second optical receiver (212B), strength of the some current signals is the same as strength of the second PPG signal, and the analog front end (220) is specifically configured to:
obtain a sixth PPG signal from the first optical receiver (212A), wherein strength of the sixth PPG signal is the same as strength of a current signal that is in the first PPG signal and that does not flow to the second optical receiver (212B); and
convert the sixth PPG signal into a voltage signal, to obtain the third PPG signal, wherein the sixth PPG signal is a current signal.

12. A method for obtaining a heart rate, applied to an electronic device (100), wherein the electronic device (100) comprises a bottom wall, at least one first optical transmitter (411), a first receiver (412), a second receiver (413), a third receiver (414), a fourth receiver (415), a first accelerometer (421), and a second accelerometer (422), the first optical transmitter (411), the first receiver (412), the second receiver (413), the third receiver (414), and the fourth receiver (415) are located on one straight line, the first receiver (412) and the second receiver (413) are distributed on one side of the first optical transmitter (411), the third receiver (414) and the fourth receiver (415) are distributed on the other side of the first optical transmitter (411), a distance between the second receiver (413) and the first optical transmitter (411) is greater than a distance between the first receiver (412) and the first optical transmitter (411), a distance between the fourth receiver (415) and the first optical transmitter (411) is greater than a distance between the third receiver (414) and the first optical transmitter (411), a spacing between the first accelerometer (421) and the fourth receiver (415) is less than a first spacing, and a spacing between the second accelerometer (422) and the second receiver (413) is less than a second spacing;
a first opening and a second opening are provided on the bottom wall, an optical signal transmitted by the first optical transmitter is transmitted from the bottom wall through the first opening, and the first receiver and the second receiver receive optical signals through the second opening;
the method comprises:
driving, by the electronic device (100), the first optical transmitter (411) to transmit a first optical signal with a first wavelength, wherein the first wavelength is greater than or equal to 492 nanometers and less than or equal to 577 nanometers;
obtaining, by the electronic device (100), a first signal, a second signal, a third signal, and a fourth signal respectively based on the first optical signal received by the first receiver (412), the second receiver (413), the third receiver (414), and the fourth receiver (415);
measuring, by the electronic device (100), a first acceleration of the electronic device (100) in a first direction by using the first accelerometer (421), and measuring a second acceleration of the electronic device (100) in the first direction by using the second accelerometer (422);
**characterized in that** the method further comprises:
comparing, by the electronic device (100), magnitudes of the first acceleration and the second acceleration, and selecting at least one signal from the first signal, the second signal, the third signal, and the fourth signal; and
obtaining, by the electronic device (100), a heart rate by using the selected at least one signal.

13. The method according to claim 12, wherein the first accelerometer (421) is on one side of the straight line, and the second accelerometer (422) is on the other side of the straight line.

14. The method according to claim 12 or 13, wherein the first direction is a direction perpendicular to the bottom wall;
the comparing, by the electronic device (100), magnitudes of the first acceleration and the second acceleration, and selecting at least one signal from the first signal, the second signal, the third signal, and the fourth signal specifically comprises:
if the magnitude of the first acceleration is greater than the magnitude of the second acceleration, selecting, by the electronic device (100), the fourth signal; or
if the magnitude of the second acceleration is greater than the magnitude of the first acceleration, selecting, by the electronic device (100), the second signal.

15. The method according to claim 12 or 13, wherein the first direction is a direction perpendicular to the bottom wall;
the comparing, by the electronic device (100), magnitudes of the first acceleration and the second acceleration, and selecting at least one signal from the first signal, the second signal, the third signal, and the fourth signal specifically comprises:
if the magnitude of the first acceleration is greater than the magnitude of the second acceleration, and a difference between the magnitude of the first acceleration and the magnitude of the second acceleration is greater than a first acceleration difference, selecting, by the electronic device (100), the fourth signal; or
if the magnitude of the second acceleration is greater than the magnitude of the first acceleration, and a difference between the magnitude of the second acceleration and the magnitude of the first acceleration is greater than the first acceleration difference, selecting, by the electronic device (100), the second signal.

## Patentansprüche

1. Verfahren zum Erhalten einer Herzfrequenz, angewandt auf eine elektronische Vorrichtung (100), wobei die elektronische Vorrichtung (100) eine Bodenwand (202), mindestens einen ersten optischen Sender (211A), mindestens einen zweiten optischen Sender (211B), mindestens einen ersten optischen Empfänger (212A) und mindestens einen zweiten optischen Empfänger (212B) umfasst, eine erste Öffnung und eine zweite Öffnung an der Bodenwand (202) bereitgestellt sind, optische Signale, die durch den ersten optischen Sender (211A) und den zweiten optischen Sender (211B) gesendet werden, von der Bodenwand (202) durch die erste Öffnung gesendet werden, der erste optische Empfänger (212A) und der zweite optische Empfänger (212B) optische Signale durch die zweite Öffnung empfangen, der erste optische Empfänger (212A) ein erstes optisches Filter beinhaltet und der zweite optische Empfänger (212B) ein zweites optisches Filter beinhaltet;
wobei das Verfahren Folgendes umfasst:
Ansteuern des ersten optischen Senders (211A) durch die elektronische Vorrichtung (100), um ein erstes optisches Signal (211a) mit einer ersten Wellenlänge zu senden, und Ansteuern des zweiten optischen Senders (211B), um ein zweites optisches Signal (211b) mit einer zweiten Wellenlänge zu senden, wobei die erste Wellenlänge größer als oder gleich 492 Nanometer und kleiner als oder gleich 577 Nanometer ist und die zweite Wellenlänge größer als oder gleich 1000 Nanometer und kleiner als oder gleich 1500 Nanometer ist;
Empfangen eines dritten optischen Signals durch die elektronische Vorrichtung (100) unter Verwendung des ersten optischen Empfängers (212A) und Erhalten eines ersten PPG-Signals basierend auf dem dritten optischen Signal, wobei das dritte optische Signal durch eine Reflexion oder Streuung des ersten optischen Signals (211a) gebildet wird und das erste optische Filter im ersten optischen Empfänger (212A) eine Reflexion oder Streuung des zweiten optischen Signals (211b) filtert;
Empfangen eines vierten optischen Signals durch die elektronische Vorrichtung (100) unter Verwendung des zweiten optischen Empfängers (212B) und Erhalten eines zweiten PPG-Signals basierend auf dem vierten optischen Signal, wobei das vierte optische Signal durch eine Reflexion oder Streuung des zweiten optischen Signals (211b) gebildet wird und das zweite optische Filter im zweiten optischen Empfänger (212B) eine Reflexion oder Streuung des ersten optischen Signals (211a) filtert;
Erhalten eines dritten PPG-Signals durch die elektronische Vorrichtung (100) durch Durchführen einer Subtraktionsverarbeitung am ersten PPG-Signal und am zweiten PPG-Signal; und
Erhalten einer Herzfrequenz durch die elektronische Vorrichtung (100) unter Verwendung des dritten PPG-Signals.

2. Verfahren nach Anspruch 1, wobei die elektronische Vorrichtung (100) ferner einen Wellenlängenmultiplexer (313) und einen Reflektor (314) umfasst, der Wellenlängenmultiplexer (313) eine erste Lichteinfallsfläche, eine zweite Lichteinfallsfläche und eine erste Lichtaustrittsfläche umfasst, die erste Lichteinfallsfläche einer Lichtaustrittsfläche des ersten optischen Senders (211A) gegenüberliegt, die zweite Lichteinfallsfläche einer Lichtaustrittsfläche des zweiten optischen Senders (211B) gegenüberliegt und das Verfahren Folgendes umfasst:
Ansteuern des ersten optischen Senders (211A) durch die elektronische Vorrichtung (100), um das erste optische Signal zur ersten Lichteinfallsfläche zu senden, und Ansteuern des zweiten optischen Senders (211B), um das zweite optische Signal zur zweiten Lichteinfallsfläche zu senden, wobei das erste optische Signal und das zweite optische Signal durch den Wellenlängenmultiplexer (313) empfangen werden; und
Durchführen einer Multiplexverarbeitung des ersten optischen Signals und des zweiten optischen Signals durch die elektronische Vorrichtung (100) unter Verwendung des Wellenlängenmultiplexers (313), um ein fünftes optisches Signal zu erhalten, wobei das fünfte optische Signal von der ersten Lichtaustrittsfläche zum Reflektor gesendet wird und von der Bodenwand gesendet wird, nachdem es durch den Reflektor reflektiert wurde.

3. Verfahren nach Anspruch 2, wobei die elektronische Vorrichtung (100) ferner einen ersten Wellenleiter, einen zweiten Wellenleiter und einen dritten Wellenleiter umfasst, wobei der erste Wellenleiter dazu konfiguriert ist, die Lichtaustrittsfläche des ersten optischen Senders (211A) mit der ersten Lichteinfallsfläche zu verbinden, wobei der zweite Wellenleiter dazu konfiguriert ist, die Lichtaustrittsfläche des zweiten optischen Senders (211B) mit der zweiten Lichteinfallsfläche zu verbinden, und wobei der dritte Wellenleiter dazu konfiguriert ist, die erste Lichtaustrittsfläche mit dem Reflektor zu verbinden, und wobei das Verfahren ferner Folgendes umfasst:
Senden des ersten optischen Signals vom ersten optischen Sender (211A) zum Wellenlängenmultiplexer (313) durch die elektronische Vorrichtung (100) unter Verwendung des ersten Wellenleiters;
Senden des zweiten optischen Signals vom zweiten optischen Sender (211B) zum Wellenlängenmultiplexer (313) durch die elektronische Vorrichtung (100) unter Verwendung des zweiten Wellenleiters; und
Senden des fünften optischen Signals vom Wellenlängenmultiplexer (313) zum Reflektor durch die elektronische Vorrichtung (100) unter Verwendung des dritten Wellenleiters.

4. Verfahren nach einem der Ansprüche 1 bis 3, wobei das Erhalten eines dritten PPG-Signals durch die elektronische Vorrichtung (100) basierend auf dem ersten PPG-Signal und dem zweiten PPG-Signal insbesondere Folgendes umfasst:
Umwandeln des ersten PPG-Signals durch die elektronische Vorrichtung (100) in ein viertes PPG-Signal und Umwandeln des zweiten PPG-Signals in ein fünftes PPG-Signal, wobei sowohl das erste PPG-Signal als auch das zweite PPG-Signal Stromsignale sind und sowohl das vierte PPG-Signal als auch das fünfte PPG-Signal Spannungssignale sind; und
Subtrahieren des fünften PPG-Signals vom vierten PPG-Signal durch die elektronische Vorrichtung (100), um das dritte PPG-Signal zu erhalten.

5. Verfahren nach einem der Ansprüche 1 bis 3, wobei der erste optische Empfänger (212A) mit dem zweiten optischen Empfänger (212B) in Reihe verbunden ist, sowohl das erste PPG-Signal als auch das zweite PPG-Signal Stromsignale sind, einige Stromsignale im ersten PPG-Signal zum zweiten optischen Empfänger (212B) fließen, die Stärke der einigen Stromsignale die gleiche ist wie die Stärke des zweiten PPG-Signals und das Verfahren Folgendes umfasst:
Erhalten eines sechsten PPG-Signals durch die elektronische Vorrichtung (100) unter Verwendung des ersten optischen Empfängers (212A), wobei die Stärke des sechsten PPG-Signals die gleiche ist wie die Stärke eines Stromsignals, das im ersten PPG-Signal enthalten ist und das nicht zum zweiten optischen Empfänger (212B) fließt; und
Umwandeln des sechsten PPG-Signals durch die elektronische Vorrichtung (100) in ein Spannungssignal, um das dritte PPG-Signal zu erhalten, wobei das sechste PPG-Signal ein Stromsignal ist.

6. Verfahren nach Anspruch 1, wobei der erste optische Empfänger (212A) und der zweite optische Empfänger (212B) ein gleicher optischer Empfänger sind, das Ansteuern des ersten optischen Senders (211A) durch die elektronische Vorrichtung (100), um ein erstes optisches Signal mit einer ersten Wellenlänge zu senden, und das Ansteuern des zweiten optischen Senders (211B), um ein zweites optisches Signal mit einer zweiten Wellenlänge zu senden, das Empfangen eines dritten optischen Signals durch die elektronische Vorrichtung (100) unter Verwendung des ersten optischen Empfängers (212A), Erhalten eines ersten PPG-Signals basierend auf dem dritten optischen Signal, das Empfangen eines vierten optischen Signals durch die elektronische Vorrichtung (100) unter Verwendung des zweiten optischen Empfängers (212B) und Erhalten eines zweiten PPG-Signals basierend auf dem vierten optischen Signal insbesondere Folgendes umfassen:
Ansteuern des ersten optischen Senders (211A) durch die elektronische Vorrichtung (100) in einer ersten Zeitperiode, um das erste optische Signal zu senden;
Erhalten des ersten PPG-Signals durch die elektronische Vorrichtung (100) basierend auf dem dritten optischen Signal, das durch den optischen Empfänger empfangen wird;
Ansteuern des zweiten optischen Senders (211B) durch die elektronische Vorrichtung (100) in einer zweiten Zeitperiode, um das zweite optische Signal zu senden; und
Erhalten des zweiten PPG-Signals durch die elektronische Vorrichtung (100) basierend auf dem vierten optischen Signal, das durch den optischen Empfänger empfangen wird, wobei ein Intervall zwischen der ersten Zeitperiode und der zweiten Zeitperiode kürzer ist als eine erste Dauer.

7. Elektronische Vorrichtung (100), umfassend eine Bodenwand, mindestens einen ersten optischen Sender (211A), mindestens einen zweiten optischen Sender (211B), mindestens einen ersten optischen Empfänger (212A), mindestens einen zweiten optischen Empfänger (212B), ein analoges Frontend (220) und einen Prozessor (230), wobei eine erste Öffnung und eine zweite Öffnung an der Bodenwand bereitgestellt sind, optische Signale, die durch den ersten optischen Sender (211A) und den zweiten optischen Sender (211B) gesendet werden, von der Bodenwand durch die erste Öffnung gesendet werden, der erste optische Empfänger (212A) und der zweite optische Empfänger (212B) optische Signale durch die zweite Öffnung empfangen, der erste optische Empfänger (212A) ein erstes optisches Filter beinhaltet und der zweite optische Empfänger (212B) ein zweites optisches Filter beinhaltet;
der erste optische Sender (211A) dazu konfiguriert ist, ein erstes optisches Signal mit einer ersten Wellenlänge zu senden, wobei die erste Wellenlänge größer als oder gleich 492 Nanometer und kleiner als oder gleich 577 Nanometer ist;
der zweite optische Sender (211B) dazu konfiguriert ist, ein zweites optisches Signal mit einer zweiten Wellenlänge zu senden, wobei die zweite Wellenlänge größer als oder gleich 1000 Nanometer und kleiner als oder gleich 1500 Nanometer ist;
der erste optische Empfänger (212A) dazu konfiguriert ist, ein drittes optisches Signal zu empfangen und ein erstes PPG-Signal basierend auf dem dritten optischen Signal zu erzeugen, wobei das dritte optische Signal durch eine Reflexion oder Streuung des ersten optischen Signals gebildet wird, und das erste optische Filter im ersten optischen Empfänger (212A) dazu konfiguriert ist, eine Reflexion oder Streuung des zweiten optischen Signals (211b) zu filtern;
der zweite optische Empfänger (212B) dazu konfiguriert ist, ein viertes optisches Signal zu empfangen und ein zweites PPG-Signal basierend auf dem vierten optischen Signal zu erzeugen, wobei das vierte optische Signal durch eine Reflexion oder Streuung des zweiten optischen Signals gebildet wird, und das zweite optische Filter im zweiten optischen Empfänger (212B) dazu konfiguriert ist, eine Reflexion oder Streuung des ersten optischen Signals (211a) zu filtern;
das analoge Frontend (220) dazu konfiguriert ist, ein drittes PPG-Signal durch Durchführen einer Subtraktionsverarbeitung am ersten PPG-Signal und am zweiten PPG-Signal zu erhalten; und der Prozessor (230) dazu konfiguriert ist, eine Herzfrequenz unter Verwendung des dritten PPG-Signals zu erhalten.

8. Elektronische Vorrichtung (100) nach Anspruch 7, wobei die elektronische Vorrichtung (100) ferner einen Wellenlängenmultiplexer (313) und einen Reflektor (314) umfasst, und der Wellenlängenmultiplexer (313) eine erste Lichteinfallsfläche, eine zweite Lichteinfallsfläche und eine erste Lichtaustrittsfläche umfasst;
die erste Lichteinfallsfläche einer Lichtaustrittsfläche des ersten optischen Senders (211A) gegenüberliegt und dazu konfiguriert ist, ein durch den ersten optischen Sender (211A) gesendetes optisches Signal zu empfangen;
die zweite Lichteinfallsfläche einer Lichtaustrittsfläche des zweiten optischen Senders (211B) gegenüberliegt und dazu konfiguriert ist, ein durch den zweiten optischen Sender (211B) gesendetes optisches Signal zu empfangen; und
der Reflektor dazu konfiguriert ist, ein von der ersten Lichtaustrittsfläche gesendetes optisches Signal zu empfangen und das von der ersten Lichtaustrittsfläche gesendete optische Signal durch die Bodenwand zu senden.

9. Elektronische Vorrichtung (100) nach Anspruch 8, wobei die elektronische Vorrichtung (100) ferner einen ersten Wellenleiter, einen zweiten Wellenleiter und einen dritten Wellenleiter umfasst;
der erste Wellenleiter dazu konfiguriert ist, die Lichtaustrittsfläche des ersten optischen Senders (211A) mit der ersten Lichteinfallsfläche zu verbinden, um das erste optische Signal zum Wellenlängenmultiplexer (313) zu senden;
der zweite Wellenleiter dazu konfiguriert ist, die Lichtaustrittsfläche des zweiten optischen Senders (211B) mit der zweiten Lichteinfallsfläche zu verbinden, um das zweite optische Signal zum Wellenlängenmultiplexer (313) zu senden; und der dritte Wellenleiter dazu konfiguriert ist, die erste Lichtaustrittsfläche mit dem Reflektor zu verbinden, um das von der ersten Lichtaustrittsfläche gesendete optische Signal zum Reflektor zu senden.

10. Elektronische Vorrichtung (100) nach einem der Ansprüche 7 bis 9, wobei das analoge Frontend (220) insbesondere zu Folgendem konfiguriert ist:
Umwandeln des ersten PPG-Signals in ein viertes PPG-Signal und Umwandeln des zweiten PPG-Signals in ein fünftes PPG-Signal, wobei sowohl das erste PPG-Signal als auch das zweite PPG-Signal Stromsignale sind und sowohl das vierte PPG-Signal als auch das fünfte PPG-Signal Spannungssignale sind; und
Subtrahieren des fünften PPG-Signals vom vierten PPG-Signal, um das dritte PPG-Signal zu erhalten.

11. Elektronische Vorrichtung (100) nach einem der Ansprüche 7 bis 9, wobei der erste optische Empfänger (212A) mit dem zweiten optischen Empfänger (212B) in Reihe verbunden ist, sowohl das erste PPG-Signal als auch das zweite PPG-Signal Stromsignale sind, einige Stromsignale im ersten PPG-Signal zum zweiten optischen Empfänger (212B) fließen, die Stärke der einigen Stromsignale die gleiche ist wie die Stärke des zweiten PPG-Signals und das analoge Frontend (220) insbesondere zu Folgendem konfiguriert ist:
Erhalten eines sechsten PPG-Signals vom ersten optischen Empfänger (212A), wobei die Stärke des sechsten PPG-Signals die gleiche ist wie die Stärke eines Stromsignals, das im ersten PPG-Signal enthalten ist und das nicht zum zweiten optischen Empfänger (212B) fließt; und
Umwandeln des sechsten PPG-Signals in ein Spannungssignal, um das dritte PPG-Signal zu erhalten, wobei das sechste PPG-Signal ein Stromsignal ist.

12. Verfahren zum Erhalten einer Herzfrequenz, angewendet auf eine elektronische Vorrichtung (100), wobei die elektronische Vorrichtung (100) eine Bodenwand, mindestens einen ersten optischen Sender (411), einen ersten Empfänger (412), einen zweiten Empfänger (413), einen dritten Empfänger (414), einen vierten Empfänger (415), einen ersten Beschleunigungsmesser (421) und einen zweiten Beschleunigungsmesser (422) umfasst, der erste optische Sender (411), der erste Empfänger (412), der zweite Empfänger (413), der dritte Empfänger (414) und der vierte Empfänger (415) auf einer geraden Linie angeordnet sind, der erste Empfänger (412) und der zweite Empfänger (413) auf einer Seite des ersten optischen Senders (411) verteilt sind, der dritte Empfänger (414) und der vierte Empfänger (415) auf der anderen Seite des ersten optischen Senders (411) verteilt sind, ein Abstand zwischen dem zweiten Empfänger (413) und dem ersten optischen Sender (411) größer ist als ein Abstand zwischen dem ersten Empfänger (412) und dem ersten optischen Sender (411), ein Abstand zwischen dem vierten Empfänger (415) und dem ersten optischen Sender (411) größer ist als ein Abstand zwischen dem dritten Empfänger (414) und dem ersten optischen Sender (411), eine Beabstandung zwischen dem ersten Beschleunigungsmesser (421) und dem vierten Empfänger (415) kleiner ist als eine erste Beabstandung, und eine Beabstandung zwischen dem zweiten Beschleunigungsmesser (422) und dem zweiten Empfänger (413) kleiner ist als eine zweite Beabstandung;
eine erste Öffnung und eine zweite Öffnung an der Bodenwand bereitgestellt sind, ein durch den ersten optischen Sender gesendetes optisches Signal von der Bodenwand durch die erste Öffnung gesendet wird, und der erste Empfänger und der zweite Empfänger optische Signale durch die zweite Öffnung empfangen; wobei das Verfahren Folgendes umfasst:
Ansteuern des ersten optischen Senders (411) durch die elektronische Vorrichtung (100), um ein erstes optisches Signal mit einer ersten Wellenlänge zu senden, wobei die erste Wellenlänge größer als oder gleich 492 Nanometer und kleiner als oder gleich 577 Nanometer ist;
Erhalten eines ersten Signals, eines zweiten Signals, eines dritten Signals und eines vierten Signals durch die elektronische Vorrichtung (100) jeweils basierend auf dem ersten optischen Signal, das durch den ersten Empfänger (412), den zweiten Empfänger (413), den dritten Empfänger (414) und den vierten Empfänger (415) empfangen wird;
Messen einer ersten Beschleunigung der elektronischen Vorrichtung (100) in einer ersten Richtung durch die elektronische Vorrichtung (100) unter Verwendung des ersten Beschleunigungsmessers (421) und Messen einer zweiten Beschleunigung der elektronischen Vorrichtung (100) in der ersten Richtung unter Verwendung des zweiten Beschleunigungsmessers (422);
**dadurch gekennzeichnet, dass** das Verfahren ferner Folgendes umfasst:
Vergleichen der Größen der ersten Beschleunigung und der zweiten Beschleunigung durch die elektronische Vorrichtung (100) und Auswählen mindestens eines Signals aus dem ersten Signal, dem zweiten Signal, dem dritten Signal und dem vierten Signal; und Erhalten einer Herzfrequenz durch die elektronische Vorrichtung (100) unter Verwendung des ausgewählten mindestens einen Signals.

13. Verfahren nach Anspruch 12, wobei sich der erste Beschleunigungsmesser (421) auf einer Seite der geraden Linie und der zweite Beschleunigungsmesser (422) auf der anderen Seite der geraden Linie befindet.

14. Verfahren nach Anspruch 12 oder 13, wobei die erste Richtung eine Richtung ist, die senkrecht zur Bodenwand verläuft;
das Vergleichen der Größen der ersten Beschleunigung und der zweiten Beschleunigung durch die elektronische Vorrichtung (100) und Auswählen mindestens eines Signals aus dem ersten Signal, dem zweiten Signal, dem dritten Signal und dem vierten Signal insbesondere Folgendes umfasst:
wenn die Größe der ersten Beschleunigung größer ist als die Größe der zweiten Beschleunigung, Auswählen des vierten Signals durch die elektronische Vorrichtung (100); oder
wenn die Größe der zweiten Beschleunigung größer ist als die Größe der ersten Beschleunigung, Auswählen des zweiten Signals durch die elektronische Vorrichtung (100).

15. Verfahren nach Anspruch 12 oder 13, wobei die erste Richtung eine Richtung ist, die senkrecht zur Bodenwand verläuft;
das Vergleichen der Größen der ersten Beschleunigung und der zweiten Beschleunigung durch die elektronische Vorrichtung (100) und Auswählen mindestens eines Signals aus dem ersten Signal, dem zweiten Signal, dem dritten Signal und dem vierten Signal insbesondere Folgendes umfasst:
wenn die Größe der ersten Beschleunigung größer ist als die Größe der zweiten Beschleunigung und eine Differenz zwischen der Größe der ersten Beschleunigung und der Größe der zweiten Beschleunigung größer ist als eine erste Beschleunigungsdifferenz, Auswählen des vierten Signals durch die elektronische Vorrichtung (100); oder
wenn die Größe der zweiten Beschleunigung größer ist als die Größe der ersten Beschleunigung und eine Differenz zwischen der Größe der zweiten Beschleunigung und der Größe der ersten Beschleunigung größer ist als die erste Beschleunigungsdifferenz, Auswählen des zweiten Signals durch die elektronische Vorrichtung (100).

## Revendications

1. Procédé d'obtention d'une fréquence cardiaque, appliqué à un dispositif électronique (100), dans lequel le dispositif électronique (100) comprend une paroi inférieure (202), au moins un premier émetteur optique (211A), au moins un second émetteur optique (211B), au moins un premier récepteur optique (212A) et au moins un second récepteur optique (212B), une première ouverture et une seconde ouverture sont prévues sur la paroi inférieure (202), des signaux optiques transmis par le premier émetteur optique (211A) et le second émetteur optique (211B) sont transmis à partir de la paroi inférieure (202) à travers la première ouverture, le premier récepteur optique (212A) et le second récepteur optique (212B) reçoivent des signaux optiques à travers la seconde ouverture, le premier récepteur optique (212A) comporte un premier filtre optique et le second récepteur optique (212B) comporte un second filtre optique ; le procédé comprend :
la commande, par le dispositif électronique (100), du premier émetteur optique (211A) pour transmettre un premier signal optique (211a) avec une première longueur d'onde, et la commande du second émetteur optique (211B) pour transmettre un deuxième signal optique (211b) avec une seconde longueur d'onde, dans lequel la première longueur d'onde est supérieure ou égale à 492 nanomètres et inférieure ou égale à 577 nanomètres, et la seconde longueur d'onde est supérieure ou égale à 1 000 nanomètres et inférieure ou égale à 1 500 nanomètres ;
la réception, par le dispositif électronique (100), d'un troisième signal optique à l'aide du premier récepteur optique (212A), et l'obtention d'un premier signal PPG sur la base du troisième signal optique, dans lequel le troisième signal optique est formé par réflexion ou diffusion du premier signal optique (211a), et le premier filtre optique dans le premier récepteur optique (212A) filtre la réflexion ou la diffusion du deuxième signal optique (211b) ;
la réception, par le dispositif électronique (100), d'un quatrième signal optique à l'aide du second récepteur optique (212B), et l'obtention d'un deuxième signal PPG sur la base du quatrième signal optique, dans lequel le quatrième signal optique est formé par réflexion ou diffusion du deuxième signal optique (211b), et le second filtre optique dans le second récepteur optique (212B) filtre la réflexion ou la diffusion du premier signal optique (211a) ;
l'obtention, par le dispositif électronique (100), d'un troisième signal PPG en réalisant un traitement de soustraction sur le premier signal PPG et le deuxième signal PPG ; et
l'obtention, par le dispositif électronique (100), d'une fréquence cardiaque à l'aide du troisième signal PPG.

2. Procédé selon la revendication 1, dans lequel le dispositif électronique (100) comprend en outre un multiplexeur par répartition en longueur d'onde (313) et un réflecteur (314), le multiplexeur par répartition en longueur d'onde (313) comprend une première surface d'incidence de la lumière, une seconde surface d'incidence de la lumière et une première surface d'émergence de la lumière, la première surface d'incidence de la lumière est opposée à une surface d'émergence de la lumière du premier émetteur optique (211A), la seconde surface d'incidence de la lumière est opposée à une surface d'émergence de la lumière du second émetteur optique (211B), et le procédé comprend :
la commande, par le dispositif électronique (100), du premier émetteur optique (211A) pour transmettre le premier signal optique à la première surface d'incidence de la lumière, et la commande du second émetteur optique (211B) pour transmettre le deuxième signal optique à la seconde surface d'incidence de la lumière, dans lequel le premier signal optique et le deuxième signal optique sont reçus par le multiplexeur par répartition en longueur d'onde (313) ; et
la réalisation, par le dispositif électronique (100), d'un traitement de multiplexage sur le premier signal optique et le deuxième signal optique à l'aide du multiplexeur par répartition en longueur d'onde (313), pour obtenir un cinquième signal optique, dans lequel le cinquième signal optique est transmis à partir de la première surface d'émergence de la lumière vers le réflecteur, et est transmis à partir de la paroi inférieure après avoir été réfléchi par le réflecteur.

3. Procédé selon la revendication 2, dans lequel le dispositif électronique (100) comprend en outre un premier guide d'ondes, un deuxième guide d'ondes et un troisième guide d'ondes, le premier guide d'ondes est configuré pour connecter la surface d'émergence de la lumière du premier émetteur optique (211A) à la première surface d'incidence de la lumière, le deuxième guide d'ondes est configuré pour connecter la surface d'émergence de la lumière du second émetteur optique (211B) à la seconde surface d'incidence de la lumière et le troisième guide d'ondes est configuré pour connecter la première surface d'émergence de la lumière au réflecteur, et le procédé comprend en outre :
la transmission, par le dispositif électronique (100), du premier signal optique à partir du premier émetteur optique (211A) au multiplexeur par répartition en longueur d'onde (313) à l'aide du premier guide d'ondes ;
la transmission, par le dispositif électronique (100), du deuxième signal optique à partir du second émetteur optique (211B) au multiplexeur par répartition en longueur d'onde (313) à l'aide du deuxième guide d'ondes ; et
la transmission, par le dispositif électronique (100), du cinquième signal optique à partir du multiplexeur par répartition en longueur d'onde (313) au réflecteur à l'aide du troisième guide d'ondes.

4. Procédé selon l'une quelconque des revendications 1 à 3, dans lequel l'obtention, par le dispositif électronique (100), d'un troisième signal PPG sur la base du premier signal PPG et du deuxième signal PPG comprend spécifiquement :
la conversion, par le dispositif électronique (100), du premier signal PPG en un quatrième signal PPG, et la conversion du deuxième signal PPG en un cinquième signal PPG, dans lequel le premier signal PPG et le deuxième signal PPG sont des signaux de courant, et le quatrième signal PPG et le cinquième signal PPG sont des signaux de tension ; et
la soustraction, par le dispositif électronique (100), du cinquième signal PPG du quatrième signal PPG, pour obtenir le troisième signal PPG.

5. Procédé selon l'une quelconque des revendications 1 à 3, dans lequel le premier récepteur optique (212A) est connecté en série au second récepteur optique (212B), le premier signal PPG et le deuxième signal PPG sont des signaux de courant, certains signaux de courant dans le premier signal PPG circulent vers le second récepteur optique (212B), l'amplitude des certains signaux de courant est la même que l'amplitude du deuxième signal PPG, et le procédé comprend :
l'obtention, par le dispositif électronique (100), d'un sixième signal PPG à l'aide du premier récepteur optique (212A), dans lequel l'amplitude du sixième signal PPG est la même que l'amplitude d'un signal de courant qui se trouve dans le premier signal PPG et qui ne circule pas vers le second récepteur optique (212B) ; et
la conversion, par le dispositif électronique (100), du sixième signal PPG en un signal de tension, pour obtenir le troisième signal PPG, dans lequel le sixième signal PPG est un signal de courant.

6. Procédé selon la revendication 1, dans lequel le premier récepteur optique (212A) et le second récepteur optique (212B) sont un récepteur optique identique, la commande, par le dispositif électronique (100), du premier émetteur optique (211A) pour transmettre un premier signal optique avec une première longueur d'onde et la commande du second émetteur optique (211B) pour transmettre un deuxième signal optique avec une seconde longueur d'onde, la réception, par le dispositif électronique (100), d'un troisième signal optique à l'aide du premier récepteur optique (212A) et l'obtention d'un premier signal PPG sur la base du troisième signal optique, et la réception, par le dispositif électronique (100), d'un quatrième signal optique à l'aide du second récepteur optique (212B) et l'obtention d'un deuxième signal PPG sur la base du quatrième signal optique comprend spécifiquement :
la commande, par le dispositif électronique (100) dans une première période de temps, du premier émetteur optique (211A) pour transmettre le premier signal optique ;
l'obtention, par le dispositif électronique (100), du premier signal PPG sur la base du troisième signal optique reçu par le récepteur optique ;
la commande, par le dispositif électronique (100) dans une seconde période de temps, du second émetteur optique (211B) pour transmettre le deuxième signal optique ; et
l'obtention, par le dispositif électronique (100), du deuxième signal PPG sur la base du quatrième signal optique reçu par le récepteur optique, dans lequel
un intervalle entre la première période de temps et la seconde période de temps est inférieur à une première durée.

7. Dispositif électronique (100), comprenant une paroi inférieure, au moins un premier émetteur optique (211A), au moins un second émetteur optique (211B), au moins un premier récepteur optique (212A), au moins un second récepteur optique (212B), une extrémité frontale analogique (220) et un processeur (230), dans lequel une première ouverture et une seconde ouverture sont prévues sur la paroi inférieure, des signaux optiques transmis par le premier émetteur optique (211A) et le second émetteur optique (211B) sont transmis à partir de la paroi inférieure à travers la première ouverture, le premier récepteur optique (212A) et le second récepteur optique (212B) reçoivent des signaux optiques à travers la seconde ouverture, le premier récepteur optique (212A) comporte un premier filtre optique et le second récepteur optique (212B) comporte un second filtre optique ;
le premier émetteur optique (211A) est configuré pour transmettre un premier signal optique avec une première longueur d'onde, dans lequel la première longueur d'onde est supérieure ou égale à 492 nanomètres et inférieure ou égale à 577 nanomètres ;
le second émetteur optique (211B) est configuré pour transmettre un deuxième signal optique avec une seconde longueur d'onde, dans lequel la seconde longueur d'onde est supérieure ou égale à 1 000 nanomètres et inférieure ou égale à 1 500 nanomètres ; le premier récepteur optique (212A) est configuré pour recevoir un troisième signal optique et générer un premier signal PPG sur la base du troisième signal optique, dans lequel le troisième signal optique est formé par réflexion ou diffusion du premier signal optique, et le premier filtre optique dans le premier récepteur optique (212A) est configuré pour filtrer la réflexion ou la diffusion du deuxième signal optique (211b) ;
le second récepteur optique (212B) est configuré pour recevoir un quatrième signal optique et générer un deuxième signal PPG sur la base du quatrième signal optique, dans lequel le quatrième signal optique est formé par réflexion ou diffusion du deuxième signal optique, et le second filtre optique dans le second récepteur optique (212A) est configuré pour filtrer la réflexion ou la diffusion du premier signal optique (211a) ;
l'extrémité frontale analogique (220) est configurée pour obtenir un troisième signal PPG en réalisant un traitement de soustraction sur le premier signal PPG et le deuxième signal PPG ; et
le processeur (230) est configuré pour obtenir une fréquence cardiaque à l'aide du troisième signal PPG.

8. Dispositif électronique (100) selon la revendication 7, dans lequel le dispositif électronique (100) comprend en outre un multiplexeur par répartition en longueur d'onde (313) et un réflecteur (314), et le multiplexeur par répartition en longueur d'onde (313) comprend une première surface d'incidence de la lumière, une seconde surface d'incidence de la lumière et une première surface d'émergence de la lumière ;
la première surface d'incidence de la lumière est opposée à une surface d'émergence de la lumière du premier émetteur optique (211A), et est configurée pour recevoir un signal optique transmis par le premier émetteur optique (211A) ;
la seconde surface d'incidence de la lumière est opposée à une surface d'émergence de la lumière du second émetteur optique (211B), et est configurée pour recevoir un signal optique transmis par le second émetteur optique (211B) ; et
le réflecteur est configuré pour recevoir un signal optique transmis à partir de la première surface d'émergence de la lumière, et transmettre, à travers la paroi inférieure, le signal optique transmis à partir de la première surface d'émergence de la lumière.

9. Dispositif électronique (100) selon la revendication 8, dans lequel le dispositif électronique (100) comprend en outre un premier guide d'ondes, un deuxième guide d'ondes et un troisième guide d'ondes ;
le premier guide d'ondes est configuré pour connecter la surface d'émergence de la lumière du premier émetteur optique (211A) à la première surface d'incidence de la lumière, pour transmettre le premier signal optique au multiplexeur par répartition en longueur d'onde (313) ;
le deuxième guide d'ondes est configuré pour connecter la surface d'émergence de la lumière du second émetteur optique (211B) à la seconde surface d'incidence de la lumière, pour transmettre le deuxième signal optique au multiplexeur par répartition en longueur d'onde (313) ; et
le troisième guide d'ondes est configuré pour connecter la première surface d'émergence de la lumière au réflecteur, pour transmettre, au réflecteur, le signal optique transmis à partir de la première surface d'émergence de la lumière.

10. Dispositif électronique (100) selon l'une quelconque des revendications 7 à 9, dans lequel l'extrémité frontale analogique (220) est spécifiquement configurée pour :
convertir le premier signal PPG en un quatrième signal PPG, et convertir le deuxième signal PPG en un cinquième signal PPG, dans lequel le premier signal PPG et le deuxième signal PPG sont des signaux de courant, et le quatrième signal PPG et le cinquième signal PPG sont des signaux de tension ; et
la soustraction du cinquième signal PPG du quatrième signal PPG, pour obtenir le troisième signal PPG.

11. Dispositif électronique (100) selon l'une quelconque des revendications 7 à 9, dans lequel le premier récepteur optique (212A) est connecté en série au second récepteur optique (212B), le premier signal PPG et le deuxième signal PPG sont des signaux de courant, certains signaux de courant dans le premier signal PPG circulent vers le second récepteur optique (212B), l'amplitude des certains signaux de courant est la même que l'amplitude du deuxième signal PPG, et l'extrémité frontale analogique (220) est spécifiquement configurée pour :
obtenir un sixième signal PPG à partir du premier récepteur optique (212A), dans lequel l'amplitude du sixième signal PPG est la même que l'amplitude d'un signal de courant qui se trouve dans le premier signal PPG et qui ne circule pas vers le second récepteur optique (212B) ; et
convertir le sixième signal PPG en un signal de tension, pour obtenir le troisième signal PPG, dans lequel le sixième signal PPG est un signal de courant.

12. Procédé pour obtenir une fréquence cardiaque, appliqué à un dispositif électronique (100), dans lequel le dispositif électronique (100) comprend une paroi inférieure, au moins un premier émetteur optique (411), un premier récepteur (412), un deuxième récepteur (413), un troisième récepteur (414), un quatrième récepteur (415), un premier accéléromètre (421) et un second accéléromètre (422), le premier émetteur optique (411), le premier récepteur (412), le deuxième récepteur (413), le troisième récepteur (414) et le quatrième récepteur (415) sont situés sur une ligne droite, le premier récepteur (412) et le deuxième récepteur (413) sont situés sur un côté du premier émetteur optique (411), le troisième récepteur (414) et le quatrième récepteur (415) sont distribués sur l'autre côté du premier émetteur optique (411), une distance entre le deuxième récepteur (413) et le premier émetteur optique (411) est supérieure à une distance entre le premier récepteur (412) et le premier émetteur optique (411), une distance entre le quatrième récepteur (415) et le premier émetteur optique (411) est supérieure à une distance entre le troisième récepteur (414) et le premier émetteur optique (411), un espacement entre le premier accéléromètre (421) et le quatrième récepteur (415) est inférieur à un premier espacement, et un espacement entre le second accéléromètre (422) et le deuxième récepteur (413) est inférieur à un second espacement ;
une première ouverture et une seconde ouverture sont prévues sur la paroi inférieure, un signal optique transmis par le premier émetteur optique est transmis à partir de la paroi inférieure à travers la première ouverture, et le premier récepteur et le deuxième récepteur reçoivent des signaux optiques à travers la seconde ouverture ;
le procédé comprend :
la commande, par le dispositif électronique (100), du premier émetteur optique (411) pour transmettre un premier signal optique avec une première longueur d'onde, dans lequel la première longueur d'onde est supérieure ou égale à 492 nanomètres et inférieure ou égale à 577 nanomètres ;
l'obtention, par le dispositif électronique (100), d'un premier signal, d'un deuxième signal, d'un troisième signal et d'un quatrième signal respectivement sur la base du premier signal optique reçu par le premier récepteur (412), le deuxième récepteur (413), le troisième récepteur (414) et le quatrième récepteur (415) ;
la mesure, par le dispositif électronique (100), d'une première accélération du dispositif électronique (100) dans une première direction à l'aide du premier accéléromètre (421), et la mesure d'une seconde accélération du dispositif électronique (100) dans la première direction à l'aide du second accéléromètre (422) ;
**caractérisé en ce que** le procédé comprend également :
la comparaison, par le dispositif électronique (100), de magnitudes de la première accélération et de la seconde accélération, et la sélection d'au moins un signal parmi le premier signal, le deuxième signal, le troisième signal et le quatrième signal ; et
l'obtention, par le dispositif électronique (100), d'une fréquence cardiaque à l'aide de l'au moins un signal sélectionné.

13. Procédé selon la revendication 12, dans lequel le premier accéléromètre (421) est sur un côté de la ligne droite et le second accéléromètre (422) est sur l'autre côté de la ligne droite.

14. Procédé selon la revendication 12 ou 13, dans lequel la première direction est une direction perpendiculaire à la paroi inférieure ;
la comparaison, par le dispositif électronique (100), de magnitudes de la première accélération et de la seconde accélération, et la sélection d'au moins un signal parmi le premier signal, le deuxième signal, le troisième signal et le quatrième signal comprend spécifiquement :
si la magnitude de la première accélération est supérieure à la magnitude de la seconde accélération, la sélection, par le dispositif électronique (100), du quatrième signal ; ou
si la magnitude de la seconde accélération est supérieure à la magnitude de la première accélération, la sélection, par le dispositif électronique (100), du deuxième signal.

15. Procédé selon la revendication 12 ou 13, dans lequel la première direction est une direction perpendiculaire à la paroi inférieure ;
la comparaison, par le dispositif électronique (100), de magnitudes de la première accélération et de la seconde accélération, et la sélection d'au moins un signal parmi le premier signal, le deuxième signal, le troisième signal et le quatrième signal comprend spécifiquement :
si la magnitude de la première accélération est supérieure à la magnitude de la seconde accélération, et qu'une différence entre la magnitude de la première accélération et la magnitude de la seconde accélération est supérieure à une première différence d'accélération, la sélection, par le dispositif électronique (100), du quatrième signal ; ou
si la magnitude de la seconde accélération est supérieure à la magnitude de la première accélération, et qu'une différence entre la magnitude de la seconde accélération et la magnitude de la première accélération est supérieure à la première différence d'accélération, la sélection, par le dispositif électronique (100), du deuxième signal.
